# FASCICULE DE BREVET EUROPEEN

(11) **EP 1 361 849 B1**
(45) Date de publication et mention de la délivrance du brevet: **12.03.2008**
(21) Numéro de dépôt: 02710955.2
(22) Date de dépôt: 08.01.2002
(51) Int. Cl.: A61K 8/60, A61K 8/81, A61Q 5/00, A61Q 19/10

(54) **COMPOSITION DE NETTOYAGE CONTENANT UN POLYMERE AMPHIPHILE**
REINIGUNGSMITTEL ENTHALTEND EIN AMPHIPHILES POLYMER
CLEANSING COMPOSITION CONTAINING AN AMPHIPHILIC POLYMER

(30) Priorité: 11.01.2001 FR 0100330
(43) Date de publication de la demande: 19.11.2003
(73) Titulaire: L'ORÉAL, 75008 Paris (FR)
(72) Inventeur: GUILLOU, Véronique, F-92160 Antony (FR); CARTON, Isabelle, 94160 Saint Mande (FR)
(74) Mandataire: Rasson, Catherine
(86) Numéro de dépôt international: PCT/FR2002/000048
(87) Numéro de publication internationale: WO 2002/055040

(56) Documents cités:
- EP-A- 0 406 042
- EP-A- 0 750 899
- EP-A- 0 815 843
- EP-A- 1 055 406
- WO-A-00/31154
- WO-A-98/56333
- US-A- 4 460 732
- US-A- 4 861 499
- US-A- 5 089 578
- US-A- 5 114 706
- US-A- 5 464 452
- A KOBAYASHI ET ALL: "Solubilization Properties of N-substituted Amphiphilic Acrylamide Copolymers" JOURNAL OF APPLIED POLYMER SCIENCE., vol. 73, no. 12, 19 septembre 1999 (1999-09-19), pages 2447-2453, XP002177425 JOHN WILEY AND SONS INC. NEW YORK., US ISSN: 0021-8995
- PATENT ABSTRACTS OF JAPAN vol. 1997, no. 02, 28 février 1997 (1997-02-28) & JP 08 252447 A (SHISEIDO CO LTD), 1 octobre 1996 (1996-10-01)

## Description

L'invention a pour objet une composition de nettoyage moussante, comprenant un tensioactif particulier et un polymère amphiphile comportant au moins un monomère à insaturation éthylénique à groupement sulfonique, sous forme libre ou partiellement ou totalement neutralisée et comprenant au moins une partie hydrophobe. L'invention se rapporte aussi aux utilisations de ladite composition dans les domaines cosmétique ou dermatologique, notamment comme produit de nettoyage ou de démaquillage de la peau, du cuir chevelu et/ou des cheveux.

Le nettoyage de la peau est très important pour le soin du visage. Il doit être le plus performant possible car les résidus gras tels que l'excès de sébum, les restes des produits cosmétiques utilisés quotidiennement et les produits de maquillage notamment les produits « waterproof » résistants à l'eau, s'accumulent dans les replis cutanés et peuvent obstruer les pores de la peau et entraîner l'apparition de boutons.

Pour le nettoyage de la peau, il est connu d'utiliser des compositions détergentes moussantes. Leur action nettoyante est apportée par les tensioactifs qu'elles contiennent, ces tensioactifs mettant en suspension les résidus gras et les pigments des produits de maquillage. Ces compositions sont efficaces et agréables à utiliser du fait qu'elles moussent, et le volume de mousse est généralement proportionnel à la concentration en tensioactifs. Toutefois, l'incorporation d'une trop grande quantité de tensioactifs peut être nuisible pour la bonne tolérance cutanée et oculaire des compositions, notamment pour les sujets à peau sensible. Ainsi, dans le domaine des nettoyants moussants, on cherche à allier à la fois performances moussantes et tolérance.

Par ailleurs, les compositions moussantes sont généralement assez fluides, ce qui rend leur manipulation parfois délicate, et il est difficile de les épaissir tout en gardant de bonnes propriétés moussantes, car l'addition d'épaississants pour augmenter la viscosité a généralement comme conséquence une diminution du volume de mousse : on dit habituellement que la viscosité « tué » la mousse.

Il subsiste donc le besoin d'une composition moussante ne comportant pas une trop grande quantité de tensioactif, et ayant une bonne qualité de mousse et une viscosité satisfaisante, tout en ayant une bonne tolérance oculaire et cutanée.

La demanderesse a découvert de manière surprenante qu'on pouvait atteindre le but de l'invention et obtenir une composition moussante ayant à la fois de bonnes propriétés cosmétiques (qualités de la mousse, viscosité satisfaisante) et de bonnes propriétés de tolérance, en utilisant une nouvelle famille de polymères amphiphiles et certains tensioactifs moussants.

Ainsi, la présente demande a pour objet une composition de nettoyage, contenant, dans un milieu aqueux physiologiquement acceptable, au moins un tensioactif choisi parmi les alkylpolyglycosides, les esters de maltose, les alcools gras glycérolés, les dérivés de N-alkylglucamine, les amido-éther carboxylates, les acétates, les alaninates, les aspartates, les glycinates, les citrates, les galacturonates, les sels d'acides gras constituant les savons, les phosphates, les tensioactifs amphotères et zwitterioniques, et au moins un polymère amphiphile comportant au moins un monomère à insaturation éthylénique à groupement sulfonique, sous forme libre ou partiellement ou totalement neutralisée et comprenant au moins une partie hydrophobe.

On entend dans la présente demande par « milieu physiologiquement acceptable » un milieu compatible avec toutes les matières kératiniques telles que la peau y compris le cuir chevelu, les ongles, les muqueuses, les yeux et les cheveux ou toute autre zone cutanée du corps. Par ailleurs, il s'agit d'un milieu aqueux, c'est-à-dire d'un milieu comportant une quantité d'eau d'au moins 30 % en poids par rapport au poids total de la composition.

La viscosité des compositions selon l'invention peut aller par exemple de 0,001 à 100 poises (0,0001 à 10 Pa.s), de préférence de 0,01 à 80 poises (0,001 à 8 Pa.s) et mieux de 1 à 60 poises (0,1 à 6 Pa.s), mesurée à environ 25°C à l'aide du Rheomat RM180 de Rheometric Scientific, cet appareil étant équipé d'un mobile différent selon les viscosités, par exemple d'un mobile 2 pour les gammes de viscosités inférieures à 7 poises, d'un mobile 3 pour les gammes de viscosités de 2 à 40 poises, et d'un mobile 4 pour les gammes de viscosités de 20 poises à 80 poises.

Les polymères amphiphiles utilisés dans la composition de l'invention, notamment ceux qui sont réticulés, permettent d'obtenir une qualité de mousse particulièrement satisfaisante, sans addition d'une trop grande quantité de tensioactif moussant, que ce tensioactif soit non ionique, anionique, amphotère ou zwitterionique. Ces polymères permettent notamment d'augmenter le volume de la mousse par rapport au volume obtenu avec le tensioactif sans polymère ou avec un polymère autre que celui utilisé conformément à la présente demande.

Ainsi, la présente invention a également pour objet l'utilisation d'un polymère amphiphile comportant au moins un monomère à insaturation éthylénique à groupement sulfonique, sous forme libre ou partiellement ou totalement neutralisée et comprenant au moins une partie hydrophobe, pour augmenter le volume de mousse d'une composition contenant au moins un tensioactif.

D'autres caractéristiques, aspects et avantages de la présente invention apparaîtront à la lecture de la description détaillée qui va suivre.

### Polymères amphiphiles selon l'invention

Les polymères conformes à l'invention sont des polymères amphiphiles comportant au moins un monomère à insaturation éthylènique à groupement sulfonique, sous forme libre ou partiellement ou totalement neutralisée et comprenant au moins une partie hydrophobe.

On entend par polymère amphiphile, tout polymère comportant à la fois une partie hydrophile et une partie hydrophobe et notamment une chaîne grasse.

La partie hydrophobe présente dans les polymères de l'invention comporte de préférence de 6 à 50 atomes de carbone, plus préférentiellement de 6 à 22 atomes de carbone, plus préférentiellement encore de 6 à 18 atomes de carbone, et plus particulièrement de 12 à 18 atomes de carbone.

De façon préférentielle, les polymères conformes à l'invention sont neutralisés partiellement ou totalement par une base minérale (soude, potasse, ammoniaque) ou une base organique telle que la mono-, di- ou tri-éthanolamine, un aminométhylpropanediol, la N-méthyl-glucamine, les acides aminés basiques comme l'arginine et la lysine, et les mélanges de ces composés.

Les polymères amphiphiles conformes à l'invention ont généralement un poids moléculaire moyen en nombre allant de 1000 à 20 000 000 g/mole, de préférence allant de 20 000 à 5 000 000 et plus préférentiellement encore de 100 000 à 1 500 000 g/mole.

Les polymères amphiphiles selon l'invention peuvent être réticulés ou non-réticulés. De préférence, on choisit des polymères amphiphiles réticulés.

Lorsqu'ils sont réticulés, les agents de réticulation peuvent être choisis parmi les composés à polyinsaturation oléfinique couramment utilisés pour la réticulation des polymères obtenus par polymérisation radicalaire.

On peut citer par exemple, le divinylbenzène, l'éther diallylique, le dipropylèneglycol-diallyléther, les polyglycol-diallyléthers, le triéthylèneglycoldivinyléther, l'hydroquinone-diallyl-éther, le di(méth)acrylate de d'éthylèneglycol ou de tétraéthylèneglycol, le triméthylol propane triacrylate, le méthylène-bis-acrylamide, le méthylène-bis-méthacrylamide, la triallylamine, le triallylcyanurate, le diallylmaléate, la tétraallyléthylènediamine, le tétra-allyloxy-éthane, le triméthylolpropane-diallyléther, le (méth)acrylate d'allyle, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.

On utilisera plus particulièrement le méthylène-bis-acrylamide, le méthacrylate d'allyle ou le triméthylol propane triacrylate (TMPTA). Le taux de réticulation variera en général de 0,01 à 10% en mole et plus particulièrement de 0,2 à 2% en mole par rapport au polymère.

Les monomères à insaturation éthylènique à groupement sulfonique sont choisis notamment parmi l'acide vinylsulfonique, l'acide styrènesulfonique, les acides (méth)acrylamido(C₁-C₂₂)alkylsulfoniques, les acides N-(C₁-C₂₂)alkyl-(méth)acrylamido(C₁-C₂₂)alkylsulfoniques comme l'acide undécyl-acrylamido-méthane-sulfonique ainsi que leurs formes partiellement ou totalement neutralisées.

Plus préférentiellement, on utilisera les acides (méth)acrylamido(C₁-C₂₂) alkylsulfoniques tels que par exemple l'acide acrylamido-méthane-sulfonique, l'acide acrylamido-éthane-sulfonique, l'acide acrylamido-propane-sulfonique, l'acide 2-acrylamido-2-méthylpropane-sulfonique, l'acide méthacrylamido-2-méthylpropane-sulfonique, l'acide 2-acrylamido-n-butane-sulfonique, l'acide 2-acrylamido-2,4,4-triméthylpentane-sulfonique, l'acide 2-méthacrylamido-dodécyl-sulfonique, l'acide 2-acrylamido-2,6-diméthyl-3-heptane-sulfonique ainsi que leurs formes partiellement ou totalement neutralisées.

Plus particulièrement, on utilisera l'acide 2-acrylamido-2-méthylpropane-sulfonique (AMPS) ainsi que ses formes partiellement ou totalement neutralisées.

Les polymères amphiphiles conformes à l'invention peuvent notamment être choisis parmi les polymères amphiphiles statistiques d'AMPS modifiés par réaction avec une n-monoalkylamine ou une di-n-alkylamine en C₆-C₂₂, et tels que ceux décrits dans la demande de brevet WO-00/31154 (faisant partie intégrante du contenu de la description). Ces polymères peuvent également contenir d'autres monomères hydrophiles éthyléniquement insaturés choisis par exemple parmi les acides (méth)acryliques, leurs dérivés alkyl substitués en β ou leurs esters obtenus avec des monoalcools ou des mono- ou poly- alkylèneglycols, les (méth)acrylamides, la vinylpyrrolidone, l'anhydride maléique, l'acide itaconique ou l'acide maléique ou les mélanges de ces composés.

Les polymères préférés de l'invention sont choisis parmi les copolymères amphiphiles d'AMPS et d'au moins un monomère hydrophobe à insaturation éthylénique comportant au moins une partie hydrophobe ayant de 6 à 50 atomes de carbone, plus préférentiellement de 6 à 22 atomes de carbone, encore plus préférentiellement de 6 à 18 atomes de carbone et plus particulièrement 12 à 18 atomes de carbone.

Ces mêmes copolymères peuvent contenir en outre un ou plusieurs monomères éthyléniquement insaturés ne comportant pas de chaîne grasse, tels que les acides (méth)acryliques, leurs dérivés alkyl substitués en β ou leurs esters obtenus avec des monoalcools ou des mono- ou poly-alkylène glycols, les (méth)acrylamides, la vinylpyrrolidone, l'anhydride maléique, l'acide itaconique ou l'acide maléique ou les mélanges de ces composés.

Ces copolymères sont décrits notamment dans la demande de brevet EP-A-750899, le brevet US-A-5089578 et dans les publications de Yotaro Morishima suivantes :
- « Self-assembling amphiphilic polyelectrolytes and their nanostructures - Chinese Journal of Polymer Science Vol. 18, N°40, (2000), 323-336 » ;
- « Miscelle formation of random copolymers of sodium 2-(acrylamido)-2-methylpropanesulfonate and a non-ionic surfactant macromonomer in water as studied by fluorescence and dynamic light scattering - Macromolecules 2000, Vol. 33, N° 10 - 3694-3704 »;
- « Solution properties of miscelle networks formed by non-ionic moieties covalently bound to an polyelectrolyte : salt effects on rheological behavior - Langmuir, 2000, Vol.16, N°12, 5324-5332 » ;
- « Stimuli responsive amphiphilic copolymers of sodium 2-(acrylamido)-2-methylpropanesulfonate and associative macromonomers - Polym. Preprint, Div. Polym. Chem. 1999, 40(2), 220-221 ».

Les monomères hydrophobes à insaturation éthylénique de ces copolymères particuliers sont choisis de préférence parmi les acrylates ou les acrylamides de formule (1) suivante : 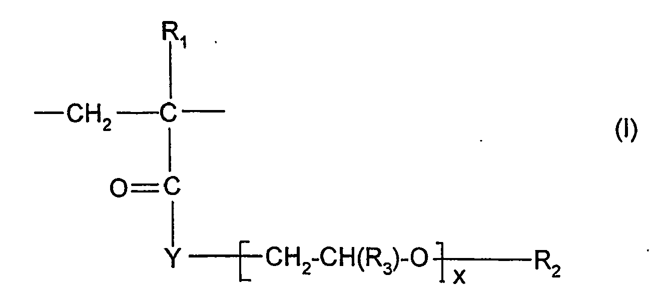 dans laquelle R₁ et R₃, identiques ou différents,'désignent un atome d'hydrogène ou un radical alkyle linéaire ou ramifié en C₁-C₆ (de préférence méthyle) ; Y désigne O ou NR₁, R₁ ayant la signification indiquée ci-dessus ; R₂ désigne un radical hydrocarboné hydrophobe comportant au moins de 6 à 50 atomes de carbone et plus préférentiellement de 6 à 22 atomes de carbone et encore plus préférentiellement de 6 à 18 atomes de carbone et plus particulièrement de 12 à 18 atomes de carbone ; x désigne un nombre de moles d'oxyde d'alkylène et varie de 0 à 100.

Le radical R₂ est choisi de préférence parmi les radicaux alkyles en C₆-C₁₈ linéaires (par exemple n-hexyle, n-octyle, n-décyle, n-hexadécyle, n-dodécyle), ramifiés ou cycliques (par exemple cyclododécane (C₁₂) ou adamantane (C₁₀)); les radicaux alkylperfluorés en C₆-C₁₈ (par exemple le groupement de formule - (CH₂)₂-(CF₂)₉-CF₃); le radical cholestéryle (C₂₇) ou un reste d'ester de cholestérol comme le groupe oxyhexanoate de cholestéryle ; les groupes polycycliques aromatiques comme le naphtalène ou le pyrène. Parmi ces radicaux, on préfère plus particulièrement les radicaux alkyles linéaires et plus particulièrement le radical n-dodécyle ou n-hexadécyle.

Selon une forme particulièrement préférée de l'invention, le monomère de formule (I) comporte au moins un motif oxyde d'alkylène (x ≥1) et de préférence une chaîne polyoxyalkylénée. La chaîne polyoxyalkylénée, de façon préférentielle, est constituée de motifs oxyde d'éthylène et/ou de motifs oxyde de propylène et encore plus particulièrement constituée de motifs oxyde d'éthylène. Le nombre de motifs oxyalkylénés (ou moles d'oxyde d'alkylène) varie en général de 3 à 100 et plus préférentiellement de 3 à 50 et encore plus préférentiellement de 7 à 25.

Parmi ces polymères, on peut citer :
- les copolymères réticulés ou non réticulés, neutralisés ou non, comportant de 15 à 60% en poids de motifs AMPS et de 40 à 85% en poids de motifs (C₈-C₁₆)alkyl(méth)acrylamide ou de motifs (C₈-C₁₆)alkyl(méth)acrylate par rapport au polymère, tels que ceux décrits dans la demande EP-A750 899 ;
- les terpolymères comportant de 10 à 90% en mole de motifs acrylamide, de 0,1 à 10% en mole de motifs AMPS et de 5 à 80% en mole de motifs n-(C₆-C₁₈)alkylacrylamide, tels que ceux décrits dans le brevet US-5089578.

On peut également citer les copolymères d'AMPS totalement neutralisé et de méthacrylate de dodécyle ou de n-hexadécyle, ainsi que les copolymères d'AMPS et de n-dodécylméthacrylamide non-réticulés et réticulés, tels que ceux décrits dans les articles de Morishima cités ci-dessus.

On citera plus particulièrement les copolymères constitués de motifs acide 2-acrylamido-2-méthylpropane-sulfonique (AMPS) de formule (II) suivante : 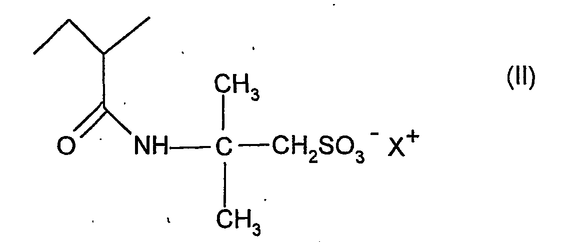 dans laquelle X⁺ est un proton, un cation de métal alcalin, un cation alcalinoterreux ou l'ion ammonium,
et de motifs de formule (III) suivante : 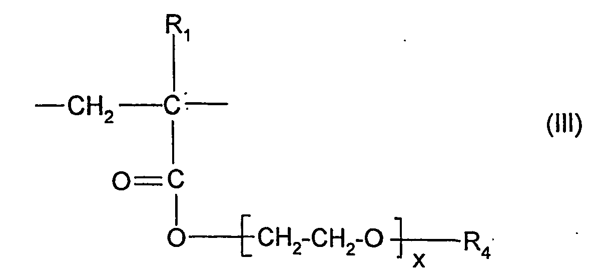 dans laquelle X désigne un nombre entier variant de 3 à 100, de préférence de 5 à 80 et plus préférentiellement de 7 à 25 R₁ a la même signification que celle indiquée ci-dessus dans la formule (1) et R₄ désigne un alkyle linéaire ou ramifié en C₆-C₂₂ et plus préférentiellement en C₁₀-C₂₂.

Les polymères particulièrement préférés sont ceux pour lesquels dans la formule (III), x = 25, R₁ désigne méthyle et R₄ représente n-dodécyle ; ils sont décrits dans les articles de Morishima mentionnés ci-dessus. D'autres polymères préférés sont ceux pour lesquels x = 25, R₁ désigne méthyle et R₄ représente n-hexadécyle ou n-octadécyle.

Les polymères pour lesquels X⁺ désigne sodium ou ammonium sont plus particulièrement préférés.

Les polymères amphiphiles préférés conformes à l'invention peuvent être obtenus selon les procédés classiques de polymérisation radicalaire en présence d'un ou plusieurs initiateurs tels que par exemple, l'azobisisobutyronitrile (AIBN), l'azobisdiméthylvaléronitrile, le chlorhydrate de 2,2-azobis-[2-amidinopropane] (ABAH=2,2-AzoBis-[2-Amidinopropane] Hydrochloride), les peroxydes organiques tels que le peroxyde de dilauryle, le peroxyde de benzoyle, l'hydroperoxyde de tert-butyle, etc..., les composés peroxydés minéraux tels que le persulfate de potassium ou d'ammonium, ou H₂O₂ éventuellement en présence de réducteurs.

Les polymères amphiphiles conformes à l'invention sont notamment obtenus par polymérisation radicalaire en milieu tert-butanol dans lequel ils précipitent. En utilisant la polymérisation par précipitation dans le tert-butanol, il est possible d'obtenir une distribution de la taille des particules du polymère particulièrement favorable pour ses utilisations.

La distribution de la taille des particules du polymère peut être déterminée par exemple par diffraction laser ou analyse d'image. Une distribution intéressante pour de type de polymère et déterminée par analyse d'image est la suivante : 60,2% inférieur à 423 microns, 52,0% inférieur à 212 microns, 26,6% inférieur à 106 microns, 2,6% inférieur à 45 microns et 26,6% supérieur à 850 microns.

La réaction peut être conduite à une température comprise entre 0 et 150°C, de préférence entre 10 et 100°C, soit à pression atmosphérique, soit sous pression réduite. Elle peut aussi être réalisée sous atmosphère inerte, et de préférence sous azote.

Selon ce procédé, on a notamment polymérisé l'acide 2-acrylamido-2-méthylpropane-sulfonique (AMPS) ou l'un de ses sels de sodium ou d'ammonium, avec un ester de l'acide (méth)acrylique et
- d'un alcool en C₁₀-C₁₈ oxyéthyléné par 8 moles d'oxyde d'éthylène (GENAPOL^{®} C-080 de la société HOECHST/CLARIANT),
- d'un alcool oxo en C₁₁ oxyéthyléné par 8 moles d'oxyde d'éthylène (GENAPOL^{®} UD-080 de la société HÔECHST/CLARIANT),
- d'un alcool en C₁₂-C₁₄ oxyéthyléné par 3 moles d'oxyde d'éthylène (GENAPOL^{®} LA-030 de la société HOECHST/CLARIANT),
- d'un alcool oxo en C₁₁ oxyéthyléné par 7 moles d'oxyde d'éthylène (GENAPOL^{®} UD-070 de la société HOECHST/CLARIANT),
- d'un alcool en C₁₂-C₁₄ oxyéthyléné par 7 moles d'oxyde d'éthylène (GENAPOL^{®} LA-070 de la société HOECHST/CLARIANT),
- d'un alcool en C₁₂-C₁₄ oxyéthyléné par 9 moles d'oxyde d'éthylène (GENAPOL^{®} LA-090 de la société HOECHST/CLARIANT),
- d'un alcool en C₁₂-C₁₄ oxyéthyléné par 11 moles d'oxyde d'éthylène (GENAPOL^{®} LA-110 de la société HOECHST/CLARIANT),
- d'un alcool en C₁₆-C₁₈ oxyéthyléné par 8 moles d'oxyde d'éthylène (GENAPOL^{®} T-080 de la société HOECHST/CLARIANT),
- d'un alcool en C₁₆-C₁₈ oxyéthyléné par 15 moles d'oxyde d'éthylène (GENAPOL^{®} T-150 de la société HOECHST/CLARIANT),
- d'un alcool en C₁₆-C₁₈ oxyéthyléné par 11 moles d'oxyde d'éthylène (GENAPOL^{®} T-110 de la société HOECHST/CLARIANT),
- d'un alcool en C₁₆-C₁₈ oxyéthyléné par 20 moles d'oxyde d'éthylène (GENAPOL^{®} T-200 de la société HOECHST/CLARIANT),
- d'un alcool en C₁₆-C₁₈ oxyéthyléné par 25 moles d'oxyde d'éthylène (GENAPOL^{®} T-250 de la société HOECHST/CLARIANT),
- d'un alcool en C₁₈-C₂₂ oxyéthyléné par 25 moles d'oxyde d'éthylène et/ou d'un alcool iso C₁₆-C₁₈ oxyéthyléné par 25 moles d'oxyde d'éthylène.

La concentration molaire en % des motifs de formule (II) et des motifs de formule (III) dans les polymères selon l'invention variera en fonction de l'application cosmétique souhaitée et des propriétés rhéologiques de la formulation recherchées. Elle peut varier entre 0,1 et 99,9% ën moles.

De préférence pour les polymères les plus hydrophobes, la proportion molaire en motifs de formule (I) ou (III) varie de 50,1 à 99,9%, plus particulièrement de 70 à 95% et encore plus particulièrement de 80 à 90%.

De préférence pour les polymères peu hydrophobes, la proportion molaire en motifs de formule (I) ou (III) varie de 0,1 à 50%, plus particulièrement de 1 à 25% et encore plus particulièrement de 3 à 20%.

La distribution des monomères dans les polymères de l'invention peut être, par exemple, alternée, bloc (y compris multibloc) ou quelconque.

Selon l'invention, on préfère que les polymères aient des chaînes pendantes sensibles à la chaleur et dont la solution aqueuse présente une viscosité qui, au delà d'une certaine température seuil, augmente, ou demeure pratiquement constante quand la température augmente.

Plus particulièrement encore, on préfère les polymères dont la solution aqueuse présente une viscosité qui est faible en dessous d'une première température seuil et qui, au dessus de cette première température seuil croît vers un maximum quand la température augmente, et qui, au dessus d'une seconde température seuil décroît à nouveau quand la température augmente. Dans cet optique, on préfère que la viscosité des solutions de polymère en dessous de la première température seuil soit de 5 à 50%, en particulier de 10 à 30% de la viscosité maximum à la seconde température seuil.

Ces polymères, de préférence, conduisent dans l'eau à un phénomène de démixion par chauffage se traduisant par des courbes présentant, en fonction de la température et de la concentration, un minimum appelé LCST (Lower Critical Solution Temperature).

Les viscosités (mesurées à 25°C au viscosimètre. Brookfield aiguille 7) des solutions aqueuses à 1 % vont de préférence de 20 000 mPa.s à 100 000 mPa.s et plus particulièrement de 60 000 mPa.s à 70 000 mPa.s.

Selon un mode préféré de réalisation de l'invention, les polymères amphiphiles utilisés dans la composition de nettoyage sont réticulés.

Les polymères amphiphiles conformes à l'invention sont présents dans les compositions dans des concentrations allant de 0,01 à 50% en poids de matière active, plus préférentiellement de 0,1 à 20%, encore plus préférentiellement de 0,2 à 10 % en poids et plus particulièrement encore de 0,25 à 5% en poids de matière active par rapport au poids total de la composition.

### Tensioactifs

Les tensioactifs utilisés dans la composition sont généralement des tensioactifs moussants, choisis parmi les alkylpolyglycosides (ou en abréviation APG), les esters de maltose, les alcools gras glycérolés, les dérivés de N-alkylglucamine, les amido-éther carboxylates, les acétates, les alaninates, les aspartates, les glycinates, les citrates, les galacturonates, les sels d'acides gras constituant les savons, les phosphates, les tensioactifs amphotères et zwitterioniques, et leurs mélanges. On peut éventuellement ajouter à ces tensioactifs, un ou plusieurs autres tensioactifs facultatifs supplémentaires choisis parmi des tensioactifs non ioniques, anioniques, et leurs mélanges.
I. Le ou les tensioactifs moussants utilisables comme tensioactifs dans la composition de l'invention peuvent être choisis parmi les tensioactifs suivants :
   1) Les alkylpolyglycosides qui peuvent être plus particulièrement représentés par la formule générale (IV) suivante :

      R-O-(G)ₓ (IV)

      dans laquelle R représente un radical alkyle linéaire ou ramifié, saturé ou insaturé, comportant de 6 à 30 atomes de carbone, G représente un sucre réduit comportant de 5 à 6 atomes de carbone, et x désigne une valeur allant de 1 à 15.
      Des alkylpolyglycosides préférés selon la présente invention sont des composés de formule (IV) dans laquelle R désigne plus particulièrement un radical alkyle comportant de 8 à 16 atomes de carbone, G désigne le glucose, le fructose ou le galactose, x est une valeur allant 1 à 4 et plus particulièrement de 1 à 3. Selon un mode préféré de réalisation de l'invention, on utilise des alkylpolyglucosides, c'est-à-dire des composés de formule (IV) où G désigne le glucose, avec x ayant de préférence une valeur allant de 1,2 à 3.
      Comme alkylpolyglucosides, on peut citer par exemple le decylglucoside (Alkyl-C9/C11-polyglucoside (1.4)) comme le produit commercialisé sous la dénomination MYDOL 10 par la société Kao Chemicals, le produit commercialisé sous la dénomination PLANTAREN 2000 UP et PLANTACARE 2000 UP par la société Henkel, et le produit commercialisé sous la dénomination ORAMIX NS10 par la société Seppic ; le caprylyl/capryl glucoside comme le produit commercialisé sous la dénomination ORAMIX CG110 par la Société Seppic ou sous la dénomination LUTENSOL GD70 par la Société BASF ; le laurylglucoside comme les produits commercialisés sous les dénominations PLANTAREN 1200 N et PLANTACARE 1200 par la société Henkel ; et le coco-glucoside comme le produit commercialisé sous la dénomination PLANTACARE 818/UP par la société Henkel, et leurs mélanges.
   2) Les dérivés de maltose qui sont par exemple ceux décrits dans le document EP-A-566438, tels que l'O-octanoyl-6'-D-maltose, ou encore le O-dodecanoyl-6'-D-maltose décrit dans le document FR-A-2,739,556.
   3) Les alcools gras polyglycérolés qui sont des alkyl éthers de polyglycéryle, avec un groupe alkyle comportant de 8 à 30 atomes de carbone et de préférence de 10 à 22 atomes de carbone. Comme alcools gras polyglycérolés, on peut citer par exemple le dodecanediol polyglycérolé (3,5 moles de glycérol) ou Polyglyceryl-3 hydroxylauryl Ether (nom CTFA) vendu sous la dénomination CHIMEXANE NF par la Société Chimex.
   4) Les dérivés de N-alkylglucamine, tels que notamment l'éthyl-2 hexyl oxy-carbonyl n-méthyl glucamine.
   5) Les amido-éthercarboxylates (AEC), tels que le Lauryl amido-éther carboxylate de sodium oxyéthyléné (3 OE) commercialisé sous la dénomination AKYPO FOAM 30® par la société KAO CHEMICALS.
   6) Les acétates tels que le 2-(2-hydroxy alkyloxy)-acétate de sodium commercialisé sous la dénomination BEAULIGHT SHAA par la société SANYO.
   7) Les alaninates comme le N-lauroyl-N methyl-amidopropionate de sodium commercialisé sous la dénomination SODIUM NIKKOL ALANINATE LN 30® par la société NIKKOL ou commercialisé sous la dénomination ALANONE ALE® par la société KAWAKEN, le N-lauroyl N-méthyl alanine triéthanolamine commercialisé sous la dénomination ALANONE ALTA ® par la société KAWAKEN.
   8) Les aspartates comme le mélange de N-lauroyl aspartate de triéthanolamine /N-myristoyl aspartate de triéthanolamine, commercialisé sous la dénomination ASPARACK® par la société MITSUBISHI.
   9) Les glycinates comme le N-cocoylglycinate de potassium ou le N-cocoylglycinate de sodium commercialisés sous les dénominations AMILITE GCS-12® et AMILITE GCK-12® par la société AJINOMOTO.
   10) Les citrates tels que le mono-ester citrique d'alcools de coco oxyéthylénés (9 OE), commercialisé sous la dénomination WITCONOL EC 1129 par la société GOLDSCHMIDT.
   11) les galacturonates tels que le Dodécyl d-galactoside uronate de sodium commercialisé par la société SOLIANCE.
   12) les sels d'acides gras constituant les savons, et par exemple ceux ayant une chaîne grasse comportant de 6 à 22 atomes de carbone, neutralisés par une base organique ou minérale telle que la potasse, la soude, et les bases organiques comme la triéthanolamine, la N-méthyl glucamine, la lysine, l'arginine. On peut citer notamment les sels de potassium et de sodium des acides gras en C10 à C22, et notamment les sels de potassium de l'acide laurique, de l'acide palmitique et de l'acide stéarique.
   13) Les phosphates, et notamment les monoalkylphosphates et les dialkylphosphates, comme par exemple le mono-phosphate de lauryle commercialisé sous la dénomination MAP 20® par la société KAO CHEMICALS, le sel de potassium de l'acide dodecyl-phosphorique, mélange mono-di-ester (majoritaire) commercialisé sous la dénomination CRAFOL AP-31® par la société PULCRA, et l'acide octyl-phosphorique, mélange mono-di-ester commercialisé sous la dénomination CRAFOL AP-20® par la société PULCRA.
   14) Les tensioactifs amphotères et zwitterioniques qui peuvent être choisis par exemple parmi les bétaïnes, les N-alkylamidobétaïnes et dérivés, les sultaïnes, les alkylpolyaminocarboxylates ; et leurs mélanges.

   Comme bétaines, on peut citer par exemple la Lauryl bétaine commercialisée sous la dénomination GENAGEN KB® par la société CLARIANT, la Lauryl bétaine oxyéthylénée (10 OE) commercialisée sous la dénomination LAURYLETHER(10 OE)BETAINE® par la société SHIN NIHON RICA, la stéaryl bétaine oxyéthylénée (10 OE) commercialisée sous la dénomination STEARYLETHER(10 OE)BETAINE® par la société SHIN NIHON RICA, la cocobétaïne commercialisée sous la dénomination EMPIGEN BB/FL par la société de ALBRIGHT & WILSON ou DEHYTON AB30 par la société COGNIS.
   Comme N-alkylamido bétaines et dérivés, on peut citer par exemple la cocamidopropyl bétaïne commercialisée sous la dénomination LEBON 2000 HG® par la société SANYO, ou commercialisé sous la dénomination EMPIGEN BB® par la société ALBRIGHT ET WILSON, la lauramidopropyl bétaine commercialisée sous la dénomination REWOTERIC AMB12P® par la société WITCO, le N-carboxyéthoxyéthyl N-cocoylamidoéthyl aminoacétate N-di-sodique, commercialisé sous la dénomination MIRANOL C2M CONCENTRE NP® par la société RHODIA CHIMIE.
   Comme sultaines, on peut citer par exemple la cocoyl amidopropyl hydroxy-sulfobetaine commercialisée sous la dénomination CROSULTAINE C-50® par la société CRODA.
   Comme alkyl polyaminocarboxylates (APAC), on peut citer par exemple le cocoyl polyaminocarboxylate de sodium, commercialisé sous la dénomination AMPHOLAK 7 CX/C®, AMPHOLAK 7 CX® par la société AKZO NOBEL, le stéaryl polyamidocarboxylate de sodium, commercialisé sous la dénomination AMPHOLAK 7 TX/C® par la société AKZO NOBEL, le carboxy-méthyl-oléyl poly propylamine de sodium, commercialisé sous la dénomination AMPHOLAK XO7/C® par la société AKZO NOBEL.
II. Les tensioactifs facultatifs supplémentaires pouvant être ajoutés dans la composition de l'invention peuvent être choisis par exemple parmi les polycondensats d'oxydes d'éthylène et de propylène sur chaine alkyle; les esters d'acides gras et de polyol ; les alkamides alkoxylés ; les sels d'acides carboxyliques polyoxyéthylénés ; les sarcosinates ; les glutamates ; les alkyl sulfates ; les alkyl éther sulfates ; les sulfonates ; les iséthionates ; les taurates ; les sulfosuccinates ; les alkylsulfoacétates ; les polypeptides ; les dérivés anioniques d'alkyl polyglucoside ; et leurs mélanges.

Comme esters d'acides gras et de polyol, on peut citer ceux ayant une chaîne grasse comportant de 8 à 30 atomes de carbone et de préférence de 10 à 22 atomes de carbone. Le polyol peut être notamment le glycérol ou les polymères de glycérol (polyglycérol) comportant plusieurs unités de glycérol. Comme esters d'acide gras et de polyol, on peut citer par exemple le monolaurate de polyglycérol, tel que le produit commercialisé sous la dénomination SUNSOFT M-12J par la société Taiyo Kagaku.

Comme alkamides alkoxylés, on peut citer notamment les alkamides éthoxylés comme le PEG-5 COCAMIDE (nom CTFA) et en particulier le produit commercialisé sous la dénomination GENAGEN CA-050 par la société Clariant.

Comme sels d'acides carboxyliques polyoxyéthylénés, on peut citer notamment le Lauryl éther carboxylate de sodium (C12-14-16 65/25/10) oxyéthyléné (6 OE) commercialisé sous la dénomination AKYPO SOFT 45 NV® par la société KAO CHEMICALS, les acides gras d'origine huile d'olive polyoxyéthylénés et carboxyméthylés, comme le produit commercialisé sous la dénomination OLIVEM 400® par la société BIOLOGIA E TECNOLOGIA, le tri-décyl éther carboxylate de sodium oxyéthyléné (6 OE) commercialisé sous la dénomination NIKKOL ECTD-6NEX® par la société NIKKOL.

Comme sarcosinates, on peut citer par exemple le Lauroyl sarcosinate de sodium commercialisé sous la dénomination SARKOSYL NL 97® par la société CIBA ou commercialisé sous la dénomination ORAMIX L 30® par la société SEPPIC, le myristoyl sarcosinate de sodium commercialisé sous la dénomination NIKKOL SARCOSINATE MN® par la société NIKKOL, le palmitoyl sarcosinate de sodium commercialisé sous la dénomination NIKKOL SARCOSINATE PN ® par la société NIKKOL.

Comme glutamates, on peut citer par exemple le mono-cocoyl glutamate de triéthanolamine commercialisé sous la dénomination ACYLGLUTAMATE CT-12® par la société AJINOMOTO, le Lauroyl-glutamate de triéthanolamine commercialisé sous la dénomination ACYLGLUTAMATE LT-12® par la société AJINOMOTO.

Comme alkylsulfates et alkyléther-sulfates, on peut citer par exemple les alkyl sulfates oxyéthylénés ou non, comme le Lauryl éther sulfate de sodium (mélange de C12-14 dans un rapport en poids 70/30) (2,2 OE), commercialisé sous la dénomination SIPON AOS 225® par la société HENKEL, le Lauryl éther sulfate d'ammonium (mélange de C12-14 dans un rapport en poids 70/30) (3 OE) commercialisé sous la dénomination SIPON LEA 370® par la société HENKEL, l'alkyl (C12-C14) éther (9 OE) sulfate d'ammonium commercialisé sous la dénomination RHODAPEX AB/20® par la société RHODIA CHIMIE., le mélange de lauryl et oléyl éther sulfate de sodium et magnesium, commercialisé sous la dénomination EMPICOL BSD 52 par la société ALBRIGHT & WILSON.

Comme sulfonates, on peut citer par exemple les alpha-oléfines sulfonates comme l'alpha oléfine sulfonate de sodium (C14-16) commercialisé sous la dénomination BIO-TERGE AS-40® par la société STEPAN, ou commercialisé sous la dénomination WITCONATE AOS PROTEGE® ou SULFRAMINE AOS PH 12® par la société WITCO ou commercialisé sous la dénomination BIO-TERGE AS-40 CG® par la société STEPAN, l'oléfine sulfonate de sodium secondaire commercialisé sous la dénomination HOSTAPUR SAS 30® par la société CLARIANT.

Comme iséthionates, on peut citer par exemple le cocoyliséthionate de sodium commercialisé sous la dénomination JORDAPON Cl P® par la société JORDAN.

Comme taurates, on peut citer par exemple le sel de sodium de méthyltaurate d'huile de palmiste, commercialisé sous la dénomination HOSTAPON CT PATE® par la société CLARIANT, le N-cocoyl N- methyltaurate de sodium commercialisé sous la dénomination HOSTAPON LT-SF® par la société CLARIANT ou commercialisé sous la dénomination NIKKOL CMT-30-T® par la société NIKKOL, le palmitoyl méthyltaurale de sodium commercialisé sous la dénomination NIKKOL PMT® par la société NIKKOL.

Comme sulfosuccinates, on peut citer par exemple le mono-sulfosuccinate d'alcool laurylique (C12/C14 70/30) oxyéthyléné (3 OE) commercialisé sous la dénomination SETACIN 103 SPECIAL® par la société ZSCHIMMER SCHWARZ ou REWOPOL SB-FA 30 K 4® par la société WITCO, le sel di-sodique d'un hemi-sulfosuccinate des alcools C12-C14, commercialisé sous la dénomination SETACIN F SPECIAL PASTE® par la société ZSCHIMMER SCHWARZ, l'oléamido sulfosuccinate di-sodique oxyéthyléné (2 OE) commercialisé sous la dénomination STANDAPOL SH 135® par la société HENKEL, le mono-sulfosuccinate d'amide laurique oxyéthyléné (5 OE) commercialisé sous la dénomination LEBON A-5000® par la société SANYO.

Comme alkyl sulfoacétates, on peut citer par exemple le mélange de lauryl sulfoacétate de sodium et de lauryl éther sulfosuccinate di-sodique, commercialisé sous la dénomination STEPAN-MILD LSB par la société STEPAN.

Comme polypeptides, on peut utiliser ceux obtenus par condensation d'une chaîne grasse sur les aminoacides du blé et de l'avoine, tels que par exemple la potassium-lauroyl proteine de blé hydrolysée, produit commercialisé sous la dénomination AMINOFOAM W OR® par la société CRODA, le sel de triéthanolamine de cocoyl proteine de soja hydrolysée, commercialisé sous la dénomination MAY-TEIN SY® par la société MAYBROOK, le sel de sodium de lauroyl amino acides d'avoine, commercialisé sous la dénomination PROTEOL OAT® par la société SEPPIC, l'hydrolysat de collagène greffé sur acide gras de coprah, commercialisé sous la dénomination GELIDERM 3000® par la société DEUTSCHE GELATINE, les protéines de soja acylées par des acides de coprah hydrogénés, produit commercialisé sous la dénomination PROTEOL VS 22® par la société SEPPIC.

Comme dérivés anioniques d'alkyl polyglucoside, on peut citer les citrates, tartrates, sulfosuccinates, carbonates et éthers de glycérol, obtenus à partir des alkyl polyglucosides, tels que par exemple le sel de sodium d'ester tartrique de cocoyl polyglucoside (1.4), commercialisé sous la dénomination EUCAROL AGE-ET® par la société CESALPINIA, le sel di-sodique d'ester sulfosuccinique de cocoyl polyglucoside (1.4), commercialisé sous la dénomination ESSAI 512 MP® par la société SEPPIC, le sel de sodium d'ester citrique de cocoyl polyglucoside (1.4) (1.4) commercialisé sous la dénomination EUCAROL AGE-EC® par la société CESALPINIA.

La composition peut contenir une quantité totale de tensioactifs (y compris avec les tensioactifs supplémentaires) allant de 0,1 à 50 % en poids de matière active, de préférence de 0,2 à 35 % et mieux de 0,5 à 20 % en poids de matière active par rapport au poids total de la composition.

Selon un mode particulier de réalisation de l'invention, la composition contient au moins comme tensioactif, un alkylpolyglycoside, et la quantité d'alkylpolyglycoside(s) représente de préférence de 0,1 à 100 % et mieux de 10 à 100 % en poids par rapport au poids total de tensioactifs.

Le milieu physiologiquement acceptable de la composition de l'invention comprend de l'eau. La quantité d'eau peut aller de 30 à 99,85 % en poids et de préférence de 30 à 95 % en poids par rapport au poids total de la composition. Ce milieu peut contenir, outre l'eau, un ou plusieurs solvants choisis parmi les alcools inférieurs comportant de 1 à 8 atomes de carbone, tels que l'éthanol ; les polyols tels que la glycérine ; les glycols comme le butylène glycol, l'isoprène glycol, le propylène glycol, les polyéthylène glycols tels que le PEG-8 ; le sorbitol ; les sucres tels que le glucose, le fructose, le maltose, le lactose, le sucrose ; et leurs mélanges. La quantité de solvant(s) dans la composition de l'invention peut aller de 0,5 à 30 % en poids et de préférence de 1 à 20 % en poids par rapport au poids total de la composition.

La composition de l'invention a de préférence un pH compatible avec la peau, c'est-à-dire allant de préférence de 3 à 9 et mieux de 4 à 8.

Les compositions de l'invention peuvent contenir des adjuvants habituellement utilisés dans le domaine cosmétique, choisis parmi les parfums, les conservateurs, les séquestrants (EDTA), les pigments, les nacres, les charges minérales ou organiques, les agents pour ajuster le pH, les colorants solubles, les filtres solaires, les actifs, les polymères cationiques, anioniques ou amphotères différents du polymère faisant l'objet de l'invention, les corps gras (huiles, alcools gras, acides gras ou cires). Les quantités de ces différents adjuvants sont celles classiquement utilisées dans le domaine considéré, et par exemple de 0,01 à 20 % du poids total de la composition. Ces adjuvants ainsi que leurs concentrations doivent être tels qu'ils ne modifient pas la propriété recherchée pour la composition de l'invention.

La composition selon l'invention peut se présenter sous toutes les formes galéniques appropriées pour une utilisation topique, et notamment sous forme de fluides, de gels, et d'émulsions H/E (phase huileuse dispersée dans phase aqueuse) ou E/H (phase aqueuse dispersée dans phase huileuse) ou d'émulsions multiples. Elle se présente de préférence sous forme de gel.

La composition de l'invention peut éventuellement contenir en outre au moins une phase huileuse comportant une ou plusieurs huiles émollientes et/ou démaquillantes.

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- des huiles essentielles naturelles ou synthétiques telles que, par exemple, les huiles d'eucalyptus, de lavandin, de lavande, de vétiver, de litsea cubeba, de citron, de santal, de romarin, de camomille, de sarriette, de noix de muscade, de cannelle, d'hysope, de carvi, d'orange, de géraniol, de cade et de bergamote;
- les alcools gras ayant de 8 à 26 atomes de carbone, comme l'alcool cétylique, l'alcool stéarylique et leur mélange (alcool cétylstéarylique), l'octyl dodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme lés phényltriméthicones, les phényldiméthicones, les phényltriméthylsiloxydiphénylsiloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.
   On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.
   Parmi les huiles indiquées ci-dessus, les huiles démaquillantes sont plus particulièrement les esters d'acide gras comportant au moins 12 atomes de carbone. Ces esters sont de préférence obtenus à partir d'un alcool à chaîne droite ou ramifiée, comportant de 1 à 17 atomes de carbone et d'un acide gras à chaîne droite ou ramifiée, comportant au moins 12 atomes de carbone et de préférence de 14 à 22 atomes de carbone. Il s'agit de préférence de mono- ou di-esters. Comme huile démaquillante, on peut citer par exemple le palmitate d'éthyl-2 hexyle (ou palmitate d'octyle), le myristate d'éthyl-2 hexyle (ou myristate d'octyle), le palmitate d'isopropyle, le myristate d'isopropyle, l'adipate de di-isopropyle, l'adipate de di-octyle, l'hexanoate d'éthyl-2 hexyle, le laurate d'éthyle, le myristate de méthyle, l'octanoate d'octyldodécyle, le néopentanoate d'isodécyle, le myristate d'éthyle, le propionate de myristyle, l'éthyl-2 hexanoate d'éthyl-2 hexyle, l'octanoate d'éthyl-2 hexyle, le caprate%aprylate d'éthyl-2 hexyle, le palmitate de méthyle, le myristate de butyle, le myristate d'isobutyle, le palmitate d'éthyle, le laurate d'isohexyle, le laurate d'hexyle, l'isostéarate d'isopropyle, et leurs mélanges.
   Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cirés comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les gommes telles que les gommes de silicone (diméthiconol); les résines de silicone telles que la trifluorométhyl-C1-4-alkyldimethicone et la trifluoropropyldimethicone ; et les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries.
   Ces corps gras peuvent être choisis de manière variée par l'homme du métier afin de préparer une composition ayant les propriétés, par exemple de consistance ou de texture, souhaitées.
   Quand elle est présente, la quantité de phase huileuse peut aller par exemple de 0,01 à 50 % en poids et de préférence de 0,1 à 30 % en poids par rapport au poids total de la composition.
   Pour obtenir des compositions plus ou moins fluides, on peut incorporer dans les compositions de l'invention outre le polymère décrit ci-dessus, un ou plusieurs agents épaississants, notamment des polymères, dans des concentrations allant par exemple de 0,05 à 10 % en poids de matière active, de préférence de 0,2 à 5 % en poids et mieux de 0,2 à 2 % en poids par rapport au poids total de la composition.
   On peut citer comme exemples d'épaississants, les sels minéraux tels que le chlorure de sodium ; les molécules oxyéthylénées et notamment les dérivés alkyl ou acyl éthoxylés de polyol qui peuvent être en particulier des dérivés oxyéthylénés d'esters d'acide gras ou d'éthers d'alcool gras et de polyol tel que glycérol, sorbitol, glucose, pentaerythritol. Comme composés de ce type, on peut citer par.exemple le stéarate de glycéryle oxyéthyléné (200 OE) tel que le produit commercialisé sous la dénomination SIMULSOL 220 TM ® par la société Seppic, le tétrastéarate de pentaérythrityle oxyéthyléné (150 OE) tel que le produit commercialisé sous la dénomination CROTHIX ® par la société Croda, le di-oléate de méthylglucose oxyéthyléné (120 OE) tel que le produit commercialisé sous la dénomination GLUCAMATE DOE-120 VEGETAL® par la société Amerchol, le triisostéarate de sorbitan oxyéthyléné (160 OE) tel que le produit commercialisé sous la dénomination RHEODOL TW IS399C par la société Kao Chemicals.
   Les compositions selon l'invention ont généralement l'apparence d'un gel ou d'une crème, éventuellement transparents. En outre, ces compositions sont stables et se rincent très bien (très bonne rinçabilité). Elles peuvent constituer notamment une composition moussante, ayant un bon volume de mousse grâce à la présence du polymère amphiphile. Ces compositions peuvent être utilisées en particulier dans les domaines cosmétique ou dermatologique, et elles peuvent constituer par exemple un produit de nettoyage et/ou de démaquillage de la peau, du cuir chevelu et/ou des cheveux, un shampooing pour les cheveux, un produit de gommage et/ou un produit exfoliant pour la peau.
   Un autre objet de l'invention consiste en l'utilisation cosmétique de la composition telle que définie ci-dessus, comme produits de nettoyage et/ou de démaquillage de la peau, du cuir chevelu et/ou des cheveux, et/ou comme shampooing, et/ou comme produit de gommage et/ou produit exfoliant pour la peau.
   Comme produit de nettoyage et/ou de démaquillage de la peau, les compositions selon l'invention peuvent être utilisées de deux façons :
   - la première utilisation consiste à étaler le gel dans les mains, à l'appliquer sur le visage ou sur le corps puis à le masser en présence d'eau pour développer la mousse directement sur le visage ou le corps.
   - l'autre utilisation possible de ce type de produit consiste à développer la mousse dans les paumes des mains avant d'être appliquée sur le visage ou le corps.

   Si la composition est suffisamment fluide, elle peut être conditionnée en flacon air spray ou aérosol automoussant. Le produit est alors délivré sous forme de mousse qui s'applique directement sur la peau ou les cheveux.
   Dans tous les cas, la mousse est ensuite rincée.
   Un autre objet de l'invention consiste en un procédé cosmétique de nettoyage de la peau, du cuir chevelu et/ou des cheveux, caractérisé par le fait qu'on applique la composition de l'invention, sur la peau, sur le cuir chevelu et/ou sur les cheveux, en présence d'eau, et qu'on élimine la mousse formée et les résidus de salissure par rinçage à l'eau.
   Les compositions selon l'invention peuvent constituer aussi une composition pour le traitement des peaux grasses (ou séborrhéiques), notamment quand elles contiennent un actif spécifique de traitement des peaux grasses tels que, comme par exemple l'acide salicylique, l'acide azélaïque, le triclosan, le piroctone olamine (octopirox), la niacinamide (vitamine PP), le panthénol (vitamine B5).
   Un autre objet de l'invention est l'utilisation de la composition telle que définie ci-dessus pour la préparation d'une composition destinée à traiter la peau grasse.

### Exemples de préparation:

### Préparation des esters (méth)acryliques éthoxylés :

Ils peuvent être notamment obtenus par action de (méth)acrylate de glycidyle, ou d'acide (méth)acrylique, ou d'un (méth)acrylate d'alkyle, ou d'un halogénure de (méth)acryloyle sur un alcool gras éthoxylé. On peut citer, à titre d'exemples non limitatifs, les préparations suivantes :
a) à partir du méthacrylate de glycidyle et du GENAPOL T-250 ;
b) à partir de l'acide (méth)acrylique et du GENAPOL UD-070 ;
c) à partir du (méth)acrylate de méthyle et du GENAPOL LA-090 ;
d) à partir du chlorure de (méth)acryloyle et du GENAPOL UD-070.

a) Dans un réacteur tricol d'un litre équipé d'un agitateur, d'un thermomètre et d'un condensateur à reflux, on introduit 500g de Genapol T-250 et 75g de méthacrylate de glycidyle. On chauffe le mélange réactionnel à la température de 100°C pendant 2 heures, et on élimine l'excès de méthacrylate de glycidyle par distillation sous pression réduite. Le monomère obtenu peut être utilisé pour la polymérisation sans purification ultérieure.
b) Dans un réacteur tricol d'un litre équipé d'un agitateur, d'un thermomètre et d'un condensateur à reflux, on introduit 500g de Genapol UD-070, 100g d'acide (méth)acrylique et de l'acide p-toluènesulfonique comme catalyseur. On chauffe le mélange réactionnel au reflux pendant 2 heures, et l'on sépare l'excès d'acide et d'eau formée au cours de la réaction par distillation sous pression réduite. Le monomère obtenu peut être utilisé pour la polymérisation sans purification ultérieure.
c) Dans un réacteur tricol d'un litre équipé d'un agitateur, d'un thermomètre et d'un condensateur a reflux, on introduit 500g de Genapol LA-090, 100g de (méth)acrylate de méthyle et 20g de tétraisopropoxyde de titane. On chauffe le mélange réactionnel au reflux pendant 2 heures, et après séparation par distillation de l'alcool formé, on distille l'ester qui reste sous pression réduite. Le monomère obtenu peut être utilisé pour la polymérisation sans purification ultérieure.
d) Dans un réacteur tricol d'un litre équipé d'un agitateur, d'un thermomètre et d'un condensateur à reflux, on introduit 500g de Genapol UD-070, 110g de chlorure de (méth)acryloyle et 50g de carbonate de sodium. On chauffe le mélange réactionnel au reflux pendant 2 heures, et l'on sépare l'excès de chlorure d'acide par distillation sous pression réduite. Le monomère obtenu peut être utilisé pour la polymérisation sans purification ultérieure.

### Polymérisation selon la méthode de précipitation dans le tert-butanol

Dans un réacteur de 2 litres muni d'un condensateur à reflux, d'une prise de gaz, d'un thermomètre et d'un agitateur, on introduit 500 ml de tert-butanol, et la quantité calculée d'AMPS. On neutralise le mélange en introduisant du NH3, et on ajoute le monomère préparé ci-dessus au mélange réactionnel. On rend le mélange réactionnel inerte par passage d'azote ou d'argon et lorsque la température intérieure a atteint 60°C, on introduit l'initiateur (AIBN) pour amorcer la polymérisation. Après quelques minutes, le polymère ainsi préparé précipite. On porte le mélange à reflux pendant 2 heures, et on sépare le polymère du solvant , par filtration à la trompe, puis on le sèche sous pression réduite:
De la façon décrite ci-dessus, on a préparé les polymères suivants :
(à partir des réactants suivants en des quantités exprimées en grammes)

| | | | | |
|---|---|---|---|---|
| Méthacrylate de Genapol T-250 | 10 | 20 | 30 | 97 |
| AMPS neutralisé par NH3 | 90 | 80 | 90 | 3 |
| Méthylène-bis-acrylamide (réticulant) | | | 1,5 | |
| Méthacrylate d'allyle (réticulant) | | 1,7 | | |
| TMPTA (réticulant) | 1,8 | | | 1,8 |
| Azobisisobutyronitrile (initiateur) | | | 1 | |
| Peroxyde dilauryle (initiateur) | 1 | 1 | | 1 |
| Tert-butanol | 300 | 300 | 300 | 300 |

### Exemples de compositions nettoyantes

Les exemples qui suivent servent à illustrer l'invention sans toutefois présenter un caractère limitatif. Les quantités indiquées sont en % en poids sauf mention contraire. (M.A. signifie de matière active)

### Exemple 1 : Gel nettoyant

- Decyl glucoside (1) 3 % M.A.
- Copolymère réticulé d'AMPS et de méthacrylate de hexadécyle 25 OE (2) 1 % M.A.
- Eau qsp 100 %

(1) APG : Alkyl (C9/11) Polyglucoside (1.4) en solution à 40%, commercialisé sous la dénomination MYDOL 10 par la société Kao Chemicals ;
(2) Copolymère réticulé par du méthacrylate d'allyle et constitué de 80% en poids de motifs AMPS neutralisés par NH₃ et de 20% en poids de motifs méthacrylate de GENAPOL T-250 [motifs de formule (III) dans laquelle R₁=CH₃, R₄=C₁₆-C₁₈ et x=25].

On a étudié les qualités sensorielles du gel obtenu par rapport à des exemples comparatifs dans lesquels le copolymère selon l'invention a été remplacé par des polymères de l'art antérieur.

Performances sensorielles : le volume de mousse développée avec le polymère amphiphile utilisé selon l'invention est évalué selon le protocole décrit ci-dessous, comparativement (1) à une composition sans polymère, (2) à une composition avec un polymère d'AMPS ne comportant pas de chaîne hydrophobe, et (3) à une composition avec un polymère carboxylique à chaîne hydrophobe.

Le protocole est le suivant : Avant toute utilisation du produit, les mains sont lavées au savon de Marseille puis convenablement rincées et séchées. Puis le protocole suivi est le suivant :
1- mouiller les mains en les passant sous l'eau courante, les secouer trois fois pour les essorer,
2- placer 1 g de produit dans le creux de l'une des mains,
3- travailler le produit entre les deux paumes pendant 10 secondes,
4- ajouter 2 ml d'eau et travailler le produit à nouveau pendant 10 secondes,
5- rincer les mains sous l'eau ,
6- les essuyer.

Le volume de mousse développé dans les mains est noté sur une échelle de 0 à 10 avant rinçage, et la note attribuée est d'autant plus élevée que le volume de la mousse est grand.

Dans le tableau ci-dessous, tous les pourcentages sont exprimés en poids de matière active (M.A.).

| Composition | Exemple 1 selon l'invention | Exemple 1 comparatif | Exemple 2 comparatif | Exemple 3 comparatif |
|---|---|---|---|---|
| Decyl glucoside (1) | 3% M.A. | 3% M.A. | 3% M.A. | 3% M.A. |
| Copolymère réticulé d'AMPS et de méthacrylate de hexadécyle 25 OE (2) | 1 % M.A. | - | - | - |
| Hostacerin AMPS (3) | - | - | 1 % M.A. | - |
| Pemulen TR2 (4) | - | - | - | 0,5 % M.A. |
| Eau | qsp 100 % | qsp 100 % | qsp 100 % | qsp 100 % |
| Viscosité (Rhéomat RM180 mobile 3, à t 10 minutes) | 24,1 UD (= 0,8 Pa.s) | 0,0001 Pa.s | 11,3 UD (= 0,3 Pa.s) | 25,5 UD (= 0,9 Pa.s) |
| Volume de mousse | 7/10 | 5,9/10 | 4,6/10 | 5,6/10 |

| | | | | |
|---|---|---|---|---|
| (1) APG : Alkyl (C9/11) Polyglucoside (1.4) en solution à 40%, commercialisé sous la dénomination MYDOL 10 par la société Kao Chemicals ; (2) Copolymère réticulé par du méthacrylate d'allyle et constitué de 80% en poids de motifs AMPS neutralisés par NH₃ et de 20% en poids de motifs méthacrylate de GENAPOL T-250 [motifs de formule (III) dans laquelle R₁=CH₃, R₄=C₁₆-C₁₈ et x=25] ; (3) Homopolymère d'acide 2-acrylamido 2-méthylpropane sulfonique (nom CTFA : Hostacerin AMPS) commercialisé par la société Clariant. (4) Copolymère acrylique à chaîne hydrophobe (nom CTFA : Acrylates/C10-30 alkyl Acrylate Crosspolymer), commercialisé par la société Goodrich). | | | | |

Il ressort du tableau ci-dessus que les compositions des exemples comparatifs donnent un volume de mousse beaucoup plus faible que l'exemple selon l'invention. De plus, le polymère selon l'invention permet d'obtenir un gel visqueux, plus visqueux qu'avec l'homopolymère d'AMPS au même taux. On obtient donc avec le polymère amphiphile utilisé dans la composition de l'invention à la fois une amélioration du volume de mousse et une bonne viscosité, ce qui est particulièrement surprenant quand on sait que l'augmentation de la viscosité par ajout d'un polymère a généralement comme conséquence une diminution du volume de mousse.

### Exemple 2 : composition nettoyante

- Decyl glucoside (1) 3 % M.A.
- Copolymère non réticulé d'AMPS (2) 1 % M.A.
- Eau qsp 100 %

(1) APG : Alkyl (C9/11) Polyglucoside (1.4) en solution à 40%, commercialisé sous la dénomination MYDOL 10 par la société Kao Chemicals ;
(2) Copolymère non réticulé, constitué de 40% en poids de motifs AMPS neutralisés par NH₃ et de 60% en poids de motifs méthacrylate de GENAPOL^{®} LA-030 [de formule (III) dans laquelle R₁=CH₃, R₄=C₁₂-C₁₄ et x=3].

Le volume de mousse obtenu avec cette composition est de 7,4/10 ; il est donc satisfaisant. Toutefois, la composition est liquide comme de l'eau (viscosité de 0,0001 Pa.s). Cet exemple montre donc que les polymères amphiphiles selon l'invention, même non réticulés, permettent d'améliorer le volume de mousse, mais que seuls les polymères réticulés permettent à la fois d'augmenter le volume de mousse et de donner une composition épaissie.

### Exemple 3 : composition nettoyante

- Cocobetaïne (1) 5 %
- Decylglucoside (2) 5 %
- Copolymère réticulé d'AMPS (3) 1 %
- Glycérine 5 %
- Conservateur qs
- Eau qsp 100 %

(1) Produit commercialisé sous la dénomination EMPIGEN BB/FL par la société Albright & Wilson;
(2) APG : Alkyl (C9/11) Polyglucoside (1.4) en solution à 40%, commercialisé sous la dénomination MYDOL 10 par la société Kao Chemicals ;
(3) Copolymère réticulé par du méthacrylate d'allyle et constitué de 80% en poids de motifs AMPS neutralisés par NH₃ et de 20% en poids de motifs méthacrylate de GENAPOL T-250 [motifs de formule (III) dans laquelle R₁=CH₃, R₄=C₁₆-C₁₈ et x=25].

On obtient un gel nettoyant onctueux, donnant une bonne qualité de mousse et agréable à utiliser.

## Revendications

1. Composition de nettoyage, contenant dans un milieu aqueux physiologiquement acceptable, au moins un tensioactif choisi parmi les alkylpolyglycosides, les esters de maltose, les alcools gras glycérolés, les dérivés de N-alkylglucamine, les amido-ether carboxylates, les acétates, les alaninates, les aspartates, les glycinates, les citrates, les galacturonates, les sels d'acides gras constituant les savons, les phosphates, les tensioactifs amphotères et zwitterioniques, et au moins un polymère amphiphile comportant au moins un' monomère à insaturation éthylénique à groupement sulfonique, sous forme libre ou partiellement ou totalement neutralisée et comprenant au moins une partie hydrophobe.

2. Composition selon la revendication 1, **caractérisée par le fait que** la partie hydrophobe du polymère amphiphile comporte de 6 à 50 atomes de carbone.

3. Composition selon la revendication précédente, **caractérisée par le fait que** la partie hydrophobe du polymère amphiphile comporte de 6 à 22 atomes de carbone.

4. Composition selon la revendication précédente, **caractérisée par le fait que** la partie hydrophobe du polymère amphiphile comporte de 6 à 18 atomes de carbone.

5. Composition selon la revendication précédente, **caractérisée par le fait que** la partie hydrophobe du polymère amphiphile comporte de 12 à 18 atomes de carbone.

6. Composition selon l'une quelconque des revendications précédentes, **caractérisée par le fait que** les polymères amphiphiles sont neutralisés partiellement ou totalement par une base minérale ou organique.

7. Composition selon l'une quelconque des revendications précédentes, **caractérisée par le fait que** les polymères amphiphiles ont un poids moléculaire moyen en nombre allant de 1000 à 20 000 000 g/mole.

8. Composition selon la revendication précédente, **caractérisée par le fait que** le poids moléculaire moyen en nombre varie de 20 000 à 5 000 000 g/mole.

9. Composition selon la revendication précédente, **caractérisée par le fait que** le poids moléculaire moyen en nombre varie de 100 000 à 1 500 000 g/mole.

10. Composition selon l'une quelconque des revendications précédentes, **caractérisée par le fait qu'**une solution aqueuse à 1 % en poids desdits polymères présente à la température de 25°C une viscosité mesurée au viscosimètre Brookfield, aiguille 7., allant de 20 000 mPa.s à 100 000 mPa.s.

11. Composition selon l'une quelconque des revendications précédentes, **caractérisée par le fait que** les polymères amphiphiles sont préparés par polymérisation radicalaire par précipitation dans le tert-butanol.

12. Composition selon l'une quelconque des revendications précédentes, **caractérisée par le fait que** les polymères amphiphiles sont réticulés ou non-réticulés.

13. Composition selon la revendication précédente, **caractérisée par le fait que** les polymères amphiphiles sont réticulés.

14. Composition selon la revendication précédente, **caractérisée par le fait que** le ou les agents de réticulation sont choisis parmi les composés à polyinsaturation oléfinique.

15. Composition selon la revendication précédente, **caractérisée par le fait que** le ou les agents de réticulation sont choisis parmi le méthylène-bis-acrylamide, le méthacrylate d'allyle ou le triméthylol propane triacrylate (TMPTA).

16. Composition selon l'une quelconque des revendications 13 à 15, **caractérisée par le fait que** le taux dé réticulation varie de préférence de 0,01 à 10% en moles et plus particulièrement de 0,2 à 2% en moles par rapport au polymère.

17. Composition selon l'une quelconque des revendications précédentes, **caractérisée par le fait que** le monomère à insaturation éthylénique à groupement sulfonique est choisi parmi l'acide vinylsulfonique, l'acide styrène sulfonique, les acides (méth)acrylamido(C₁-C₂₂)alkylsulfoniques, les acides N-(C₁-C₂₂)alkyl(méth)acrylamido(C₁-C₂₂)alkylsulfoniques ainsi que leurs formes partiellement ou totalement neutralisées.

18. Composition selon la revendication précédente, **caractérisée par le fait que** le monomère à insaturation éthylénique à groupement sulfonique est choisi parmi l'acide acrylamido-méthane-sulfonique, l'acide acrylamido-éthane-sulfonique, l'acide acrylamido-propane-sulfonique, l'acide 2-acrylamido-2-méthylpropane-sulfonique, l'acide méthacrylamido-2-méthylpropane-sulfonique, l'acide 2-acrylamido-n-butane-sulfonique, l'acide 2-acrylamido 2,4,4-triméthylpentane-sulfonique, l'acide 2-méthacrylamido-dodécyl-sulfonique, l'acide 2-acrylamido-2,6-diméthyl-3-heptane-sulfonique, ainsi que leurs formes partiellement ou totalement neutralisées.

19. Composition selon l'une quelconque des revendications 17 ou 18, **caractérisée par le fait que** le monomère à insaturation éthylénique à groupement sulfonique est l'acide 2-acrylamido-2-méthylpropane-sulfonique (AMPS), ainsi que ses formes partiellement ou totalement neutralisées.

20. Composition selon la revendication précédente, **caractérisée par le fait que** les polymères amphiphiles sont choisis parmi les polymères statistiques d'AMPS modifiés par réaction avec une n-mono(C₆-C₂₂)alkylamine ou une di-n-(C₆-C₂₂)alkylamine.

21. Composition selon l'une quelconque des revendications 19 ou 20, **caractérisée par le fait que** les polymères amphiphiles d'AMPS contiennent en plus au moins un monomère à insaturation éthylénique ne comportant pas de chaîne grasse.

22. Composition selon la revendication précédente, **caractérisée par le fait que** le monomère à insaturation éthylénique ne comportant pas de chaîne grasse est choisi parmi les acides (méth)acryliques et leurs dérivés alkyl substitués en β, et leurs esters obtenus avec des monoalcools ou des mono- ou polyalkylèneglycols, ou bien parmi les (méth)acrylamides, la vinylpyrrolidone, l'anhydride maléique, l'acide itaconique ou l'acide maléique, ou les mélanges de ces composés.

23. Composition selon l'une quelconque des revendications précédentes, **caractérisée par le fait que** les polymères amphiphiles sont choisis parmi les copolymères amphiphiles d'AMPS et d'au moins un monomère hydrophobe à insaturation éthylénique comportant au moins une partie hydrophobe ayant de 6 à 50 atomes de carbone.

24. Composition selon la revendication précédente, **caractérisée par le fait que** la partie hydrophobe comporte de 6 à 22 atomes de carbone.

25. Composition selon la revendication précédente, **caractérisée par le fait que** la partie hydrophobe comporte de 6 à 18 atomes de carbone.

26. Composition selon la revendication précédente, **caractérisée par le fait que** la partie hydrophobe comporte de 12 à 18 atomes de carbone.

27. Composition selon l'une quelconque des revendications 23 à 26, **caractérisée par le fait que** le monomère hydrophobe à insaturation éthylénique est choisi parmi les acrylates ou les acrylamides de formule (1) suivante : 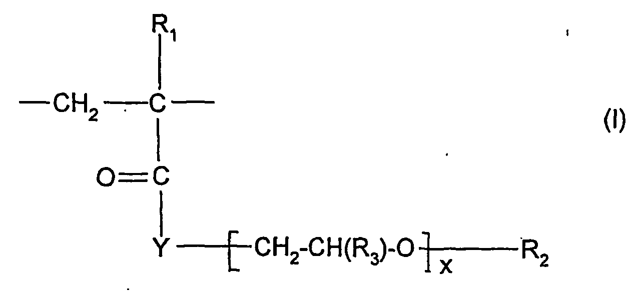 dans laquelle R₁ et R₃, identiques ou différents, désignent un atome d'hydrogène ou un radical alkyle linéaire ou ramifié en C₁-C₆ (de préférence méthyle) ; Y désigne O ou N R₁, R₁ ayant la signification indiquée ci-dessus ; R₂ désigne ,un radical hydrocarboné hydrophobe comportant au moins de 6 à 50 atomes de carbone et plus préférentiellement de 6 à 22 atomes de carbone et encore plus préférentiellement de 6 à 18 atomes de carbone et plus particulièrement de 12 à 18 atomes de carbone ; x désigne un nombre de moles d'oxyde d'alkylène et varie de 0 à 100.

28. Composition selon la revendication précédente, **caractérisée par le fait que** le radical hydrophobe R₂ est choisi parmi les radicaux alkyles en C₆-C₁₈, linéaires, ramifiés ou cycliques ; les radicaux alkylperfluorés en C₆-C₁₈; le radical cholestéryle ou un ester de cholestérol ; les groupes polycycliques aromatiques.

29. Composition selon l'une quelconque des revendications 27 ou 28 **caractérisée par le fait que** le monomère de formule (I) comporte en plus au moins un motif oxyde d'alkylène (x ≥ 1).

30. Composition selon l'une quelconque des revendications 27 à 29, **caractérisée par le fait que** le monomère de formule (I) comporte en plus au moins une chaîne polyoxyalkylénée.

31. Composition selon la revendication précédente, **caractérisée par le fait que** la chaîne polyoxyalkylénée est constituée de motifs oxyde d'éthylène et/ou de motifs oxyde de propylène.

32. Composition selon la revendication précédente, **caractérisée par le fait que** la chaîne polyoxyalkylénée est constituée uniquement de motifs oxyde d'éthylène.

33. Composition selon l'une quelconque des revendications 27 à 32, **caractérisée par le fait que** le nombre de moles d'oxyde d'alkylène varie de 3 à 100. ,

34. Composition selon la revendication précédente, **caractérisée par le fait que** le nombre de moles d'oxyde d'alkylène varie de 3 à 50.

35. Composition selon la revendication précédente, **caractérisée par le fait que** le nombre de moles d'oxyde d'alkylène varie de 7 à 25.

36. Composition selon l'une quelconque des revendications 23 à 28, **caractérisée par le fait que** le polymère amphiphile d'AMPS est choisi parmi :
- les copolymères réticulés ou non réticulés, neutralisés ou non, comportant de 15 à 60% en poids de motifs AMPS et de 40 à 85% en poids de motifs (C₈-C₁₆)alkyl(méth)acrylamide ou de motifs (C₈-C₁₆)alkyl(meth)acrylate, par rapport au polymère ;
- les terpolymères comportant de 10 à 90% en mole de motifs acrylamide, de 0,1 à 10% en mole de motifs AMPS et de 5 à 80% en mole de motifs, n-(C₆₋₁₈)alkylacrylamide, par rapport au polymère.

37. Composition selon l'une quelconque des revendications 23 à 28, **caractérisée par le fait que** le polymère amphiphile d'AMPS est choisi parmi :
- les copolymères non réticulés d'AMPS partiellement ou totalement neutralisé et de méthacrylate de n-dodécyle ou de n-hexadécyle.
- les copolymères réticulés ou non réticulés d'AMPS partiellement ou totalement neutralisé et de n-dodécylméthacrylamide.

38. Composition selon les revendications 23 à 35, **caractérisée par le fait que** le polymère amphiphile d'AMPS est choisi parmi les copolymères constitués de motifs acide 2-acrylamido-2-méthylpropane-sulfonique (AMPS) de formule (II) suivante : 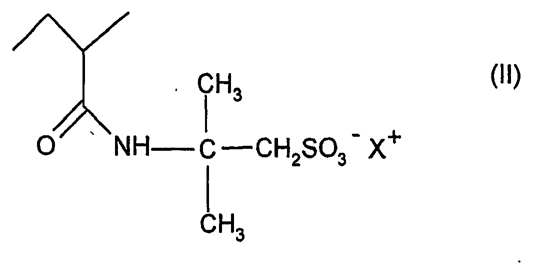 dans laquelle X⁺ est un proton, un cation de métal alcalin, un cation alcalino-terreux ou l'ion ammonium,
et de motifs de formule (III) suivante : 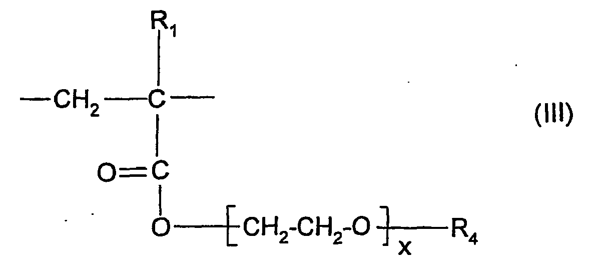 dans laquelle x désigne un nombre entier variant de 3 à 100, de préférence de 5 à 80 et plus préférentiellement de 7 à 25 ; R₁ a la même signification que celle indiquée ci-dessus dans la formule (I) et R₄ désigne un alkyle linéaire ou ramifié en C₆-C₂₂ et plus préférentiellement en C₁₀-C₂₂.

39. Composition selon la revendication précédente, **caractérisée par le fait que** x = 25, R₁ est méthyle et R₄ est n-dodécyle, n-hexadécyle ou n-octadécyle.

40. Composition selon la revendication 27 ou 38, **caractérisée par le fait que** la proportion molaire en % des motifs de formule (I) ou des motifs de formule (III) dans les polymères varie de 50,1 à 99,9%.

41. Composition selon la revendication 27 ou 38, **caractérisée par le fait que** la proportion molaire en % des motifs de formule (I) ou des motifs de formule (III) dans les polymères varie de 0,1 à 50%.

42. Composition selon l'une quelconque des revendications précédentes, **caractérisée par le fait que** la quantité de polymère(s) amphiphile(s) va de 0,01 à 50 % en poids de matière active, préférentiellement de 0,1 à 20 % en poids, plus préférentiellement de 0,2 à 10 % en poids, et plus particulièrement encore de 0,25 à 5 % en poids de matière active par rapport au poids total de la composition.

43. Composition selon l'une quelconque des revendications précédentes, **caractérisée par le fait que** le tensioactif est un alkylpolyglycoside représenté par la formule générale (IV) suivante :
R-O-(G)ₓ (IV)
dans laquelle R représente un radical alkyle linéaire ou ramifié, saturé ou insaturé, comportant de 6 à 30 atomes de carbone, G représente un sucre réduit comportant de 5 à 6 atomes de carbone, et x désigne une valeur allant de 1 à 15.

44. Composition selon la revendication précédente, **caractérisée par le fait que** l'alkylpolyglycoside est un composé de formule (IV) dans laquelle R désigne un radical alkyle comportant de 8 à 16 atomes de carbone, G désigne le glucose, le fructose ou le galactose, x est une valeur allant 1 à 4 et plus particulièrement de 1 à 3.

45. Composition selon l'une quelconque des revendications précédentes, **caractérisée par le fait que** le tensioactif est un alkylpolyglucoside.

46. Composition selon l'une quelconque des revendications précédentes, **caractérisée par le fait qu'**elle comprend en outre un ou plusieurs tensioactifs supplémentaires choisis parmi les polycondensats d'oxydes d'éthylène et de propylène sur chaine alkyle ; les esters d'acides gras et de polyol ; les alkamides alkoxylés, les sels d'acides carboxyliques polyoxyéthylénés, les sarcosinates, les glutamates, les alkyl sulfates, les alkyl éther sulfates, les sulfonates, les iséthionates, les taurates, les sulfosuccinates, les alkylsulfoacétates, les polypeptides, les dérivés anioniques d'alkyl polyglucoside, et leurs mélanges.

47. Composition selon l'une quelconque des revendications précédentes, **caractérisée par le fait que** la quantité totale de tensioactifs(s) va de 0,1 à 50 % en poids de matière active, et de préférence de 0,2 à 40 % en poids de matière active, par rapport au poids total de la composition.

48. Composition selon l'une quelconque des revendications précédentes, **caractérisée par le fait qu'**elle comprend au moins un alkylpolylglycoside.

49. Composition selon la revendications précédente, **caractérisée par le fait que** la quantité d'alkylpolyglycoside(s) représente de préférence de 0,1 à 100 % et mieux de 10 à 100 % en poids par rapport au poids total de tensioactifs.

50. Composition selon l'une quelconque des revendications précédentes, **caractérisée par le fait que** le milieu physiologiquement acceptable est constitué d'eau ou d'eau et d'au moins un solvant organique choisi dans le groupe constitué par les solvants organiques hydrophiles, les solvants organiques lipophiles, les solvants amphiphiles ou leurs mélanges.

51. Composition selon l'une quelconque des revendications précédentes, **caractérisée par le fait qu'**elle comprend en plus au moins une phase huileuse.

52. Utilisation cosmétique d'une composition selon l'une quelconque des revendications précédentes, comme produits de nettoyage et/ou de démaquillage de la peau, du cuir chevelu et/ou des cheveux et/ou comme shampooing et/ou comme produit de gommage et/ou produit exfoliant pour la peau.

53. Procédé cosmétique de nettoyage de la peau, du cuir chevelu et/ou des cheveux, **caractérisé en ce qu'**on applique la composition selon l'une quelconque des revendications 1 à 51, sur la peau, sur le cuir chevelu et/ou sur les cheveux, en présence d'eau, et qu'on élimine la mousse formée et les résidus de salissure par rinçage à l'eau.

54. Utilisation de la composition selon l'une quelconque des revendications 1 à 51, pour la préparation d'une composition destinée à traiter la peau grasse.

55. Utilisation d'un polymère amphiphile comportant au moins un monomère à insaturation éthylénique à groupement sulfonique, sous forme libre ou partiellement ou totalement neutralisée et comprenant au moins une partie hydrophobe, pour augmenter le volume de mousse d'une composition contenant au moins un tensioactif.

## Claims

1. Cleansing composition containing, in a physiologically acceptable aqueous medium, at least one surfactant chosen from alkylpolyglycosides, maltose esters, glycerolated fatty alcohols, N-alkylglucamine derivatives, amido ether carboxylates, acetates, alaninates, aspartates, glycinates, citrates, galacturonates, fatty acid salts constituting soaps, phosphates, amphoteric and zwitterionic surfactants, and at least one amphiphilic polymer comprising at least one ethylenically unsaturated monomer containing a sulfonic group in free form or in partially or totally neutralized form and comprising at least one hydrophobic part.

2. Composition according to Claim 1, **characterized in that** the hydrophobic part of the amphiphilic polymer contains from 6 to 50 carbon atoms.

3. Composition according to the preceding claim, **characterized in that** the hydrophobic part of the amphiphilic polymer contains from 6 to 22 carbon atoms.

4. Composition according to the preceding claim, **characterized in that** the hydrophobic part of the amphiphilic polymer contains from 6 to 18 carbon atoms.

5. Composition according to the preceding claim, **characterized in that** the hydrophobic part of the amphiphilic polymer contains from 12 to 18 carbon atoms.

6. Composition according to any one of the preceding claims, **characterized in that** the amphiphilic polymers are partially or totally neutralized with a mineral or organic base.

7. Composition according to any one of the preceding claims, **characterized in that** the amphiphilic polymers have a number-average molecular weight ranging from 1000 to 20 000 000 g/mol.

8. Composition according to the preceding claim, **characterized in that** the number-average molecular weight ranges from 20 000 to 5 000 000 g/mol.

9. Composition according to the preceding claim, **characterized in that** the number-average molecular weight ranges from 100 000 to 1 500 000 g/mol.

10. Composition according to any one of the preceding claims, **characterized in that** an aqueous solution containing 1% by weight of the said polymers has, at a temperature of 25°C, a viscosity, measured using a Brookfield viscometer with a No. 7 needle, ranging from 20 000 mPa.s to 100 000 mPa.s.

11. Composition according to any one of the preceding claims, **characterized in that** the amphiphilic polymers are prepared by free-radical precipitation polymerization in tert-butanol.

12. Composition according to any one of the preceding claims, **characterized in that** the amphiphilic polymers are crosslinked or non-crosslinked.

13. Composition according to the preceding claim, **characterized in that** the amphiphilic polymers are crosslinked.

14. Composition according to the preceding claim, **characterized in that** the crosslinking agent (s) is (are) chosen from compounds containing olefinic polyunsaturation.

15. Composition according to the preceding claim, **characterized in that** the crosslinking agent(s) is (are) chosen from methylenebisacrylamide, allyl methacrylate and trimethylolpropane triacrylate (TMPTA).

16. Composition according to any one of Claims 13. to 15, **characterized in that** the degree of crosslinking ranges preferably from 0.01 mol% to 10 mol% and more particularly from 0.2 mol% to 2 mol% relative to the polymer.

17. Composition according to any one of the preceding claims, **characterized in that** the ethylenically unsaturated monomer containing a sulfonic group is chosen from vinylsulfonic acid, styrenesulfonic acid, (meth) acrylamido (C₁-C₂₂) alkylsulfonic acids, N-(C₁-C₂₂)alkyl(meth)acrylamido-(C₁-C₂₂)alkylsulfonic acids, and also partially or totally neutralized forms thereof.

18. Composition according to the preceding claim, **characterized in that** the ethylenically unsaturated monomer containing a sulfonic group is chosen from acrylamidomethanesulfonic acid, acrylamidoethanesulfonic acid, acrylamidopropanesulfonic acid, 2-acrylamido-2-methylpropanesulfonic acid, methacrylamido-2-methylpropanesulfonic acid, 2-acrylamido-n-butanesulfonic acid, 2-acrylamido-2,4,4-trimethylpentanesulfonic acid, 2-methacrylamidododecylsulfonic acid and 2-acrylamido-2,6-dimethyl-3-heptanesulfonic acid, and also partially or totally neutralized forms thereof.

19. Composition according to either of Claims 17 and 18, **characterized in that** the ethylenically unsaturated monomer containing a sulfonic group is 2-acrylamido-2-methylpropanesulfonic acid (AMPS), and also partially or totally neutralized forms thereof.

20. Composition according to the preceding claim, **characterized in that** the amphiphilic polymers are chosen from random polymers of AMPS modified by reaction with an n-mono (C₆-C₂₂) alkylamine or a di-n-(C₆-C₂₂) alkylamine.

21. Composition according to either of Claims 19 and 20, **characterized in that** the amphiphilic polymers of AMPS also contain at least one ethylenically unsaturated monomer not comprising a fatty chain.

22. Composition according to the preceding claim, **characterized in that** the ethylenically unsaturated monomer not comprising a fatty chain is chosen from (meth)acrylic acids and β-substituted alkyl derivatives thereof, and esters thereof obtained with monoalcohols or mono- or polyalkylene glycols, or alternatively from (meth)acrylamides, vinylpyrrolidone, maleic anhydride, itaconic acid or maleic acid, or mixtures of these compounds.

23. Composition according to any one of the preceding claims, **characterized in that** the amphiphilic polymers are chosen from amphiphilic copolymers of AMPS and of at least one ethylenically unsaturated hydrophobic monomer comprising at least one hydrophobic part having from 6 to 50 carbon atoms.

24. Composition according to the preceding claim, **characterized in that** the hydrophobic part contains from 6 to 22 carbon atoms.

25. Composition according to the preceding claim, **characterized in that** the hydrophobic part contains from 6 to 18 carbon atoms.

26. Composition according to the preceding claim, **characterized in that** the hydrophobic part contains from 12 to 18 carbon atoms.

27. Composition according to any one of Claims 23 to 26, **characterized in that** the ethylenically unsaturated hydrophobic monomer is chosen from the acrylates or acrylamides of formula (I) below: 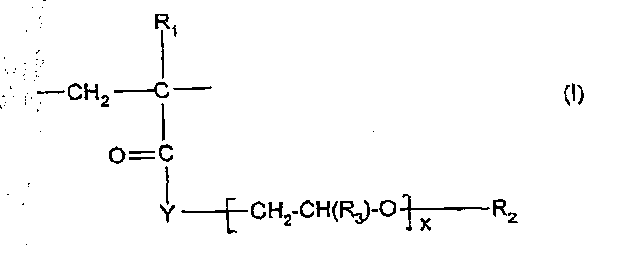 in which R₁ and R₃, which may be identical or different, denote a hydrogen atom or a linear or branched C₁-C₆ alkyl radical (preferably methyl); Y denotes O or NR₁, R₁ having the meaning given above; R₂ denotes a hydrophobic hydrocarbon-based radical containing at least from 6 to 50 carbon atoms, more preferably from 6 to 22 carbon atoms, even more preferably from 6 to 18 carbon atoms and more particularly from 12 to 18 carbon atoms; x denotes a number of moles of alkylene oxide and ranges from 0 to 100.

28. Composition according to the preceding claim, **characterized in that** the hydrophobic radical R₂ is chosen from linear, branched or cyclic C₆-C₁₈ alkyl radicals; C₆-C₁₈ alkylperfluoro radicals; the cholesteryl radical or a cholesterol ester; aromatic polycyclic groups.

29. Composition according to either of Claims 27 and 28, **characterized in that** the monomer of formula (I) also comprises at least one alkylene oxide unit (x ≥ 1).

30. Composition according to any one of Claims 27 to 29, **characterized in that** the monomer of formula (I) also comprises at least one polyoxyalkylenated chain.

31. Composition according to the preceding claim, **characterized in that** the polyoxyalkylenated chain is constituted of ethylene oxide units and/or of propylene oxide units.

32. Composition according to the preceding claim, **characterized in that** the polyoxyalkylenated chain is solely constituted of ethylene oxide units.

33. Composition according to any one of Claims 27 to 32, **characterized in that** the number of moles of alkylene oxide ranges from 3 to 100.

34. Composition according to the preceding claim, **characterized in that** the number of moles of alkylene oxide ranges from 3 to 50.

35. Composition according to the preceding claim, **characterized in that** the number of moles of alkylene oxide ranges from 7 to 25.

36. Composition according to any one of Claims 23 to 28, **characterized in that** the amphiphilic polymer of AMPS is chosen from:
- crosslinked or non-crosslinked, neutralized or non-neutralized copolymers comprising from 15% to 60% by weight of AMPS units and from 40% to 85% by weight of (C₈-C₁₆)alkyl(meth)acrylamide units or of (C₈-C₁₆) alkyl (meth) acrylate units relative to the polymer;
- terpolymers comprising from 10 mol% to 90 mol% of acrylamide units, from 0.1 mol% to 10 mol% of AMPS units and from 5 mol% to 80 mol% of n-(C₆-C₁₈)alkylacrylamide units relative to the polymer.

37. Composition according to any one of Claims 23 to 28, **characterized in that** the amphiphilic polymer of AMPS is chosen from:
- non-crosslinked copolymers of partially or totally neutralized AMPS and of n-dodecyl or n-hexadecyl methacrylate;
- crosslinked or non-crosslinked copolymers of partially or totally neutralized AMPS and of n-dodecylmethacrylamide.

38. Composition according to Claims 23 to 35, **characterized in that** the amphiphilic polymer of AMPS is chosen from copolymers constituted of 2-acrylamido-2-methylpropanesulfonic acid (AMPS) units of formula (II) below: 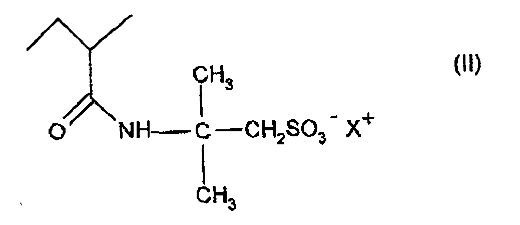 in which X⁺ is a proton, an alkali metal cation, an alkaline-earth metal cation or the ammonium ion, and of units of formula (III) below: 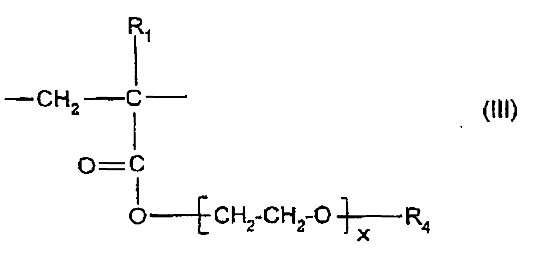 in which x denotes an integer ranging from 3 to 100, preferably from 5 to 80 and more preferably from 7 to 25; R₁ has the same meaning as that given above in formula (I) and R₄ denotes a linear or branched C₆-C₂₂ and more preferably C₁₀-C₂₂ alkyl.

39. Composition according to the preceding claim, **characterized in that** x=25, R₁ is methyl and R₄ is n-dodecyl, n-hexadecyl or n-octadecyl.

40. Composition according to Claim 27 or 38, **characterized in that** the % molar proportion of units of formula (I) or of units of formula (III) in the polymers ranges from 50.1% to 99.9%.

41. Composition according to Claim 27 or 38, **characterized in that** the % molar proportion of units of formula (I) or of units of formula (III) in the polymers ranges from 0.1% to 50%.

42. Composition according to any one of the preceding claims, **characterized in that** the amount of amphiphilic polymer(s) ranges from 0.01% to 50% by weight of active material, preferably from 0.1% to 20% by weight, more preferably from 0.2% to 10% by weight and even tore particularly from 0.25% to 5% by weight of active material relative to the total weight of the composition.

43. Composition according to any one of the preceding claims, **characterized in that** the surfactant is an alkylpolyglycoside represented by the general formula (IV) below:
R-O-(G)ₓ (IV)
in which R represents a linear or branched, saturated or unsaturated alkyl radical containing from 6 to 30 carbon atoms, G represents a reduced sugar containing from 5 to 6 carbon atoms, and x denotes a value ranging from 1 to 15.

44. Composition according to the preceding claim, **characterized in that** the alkylpolyglycoside is a compound of formula (IV) in which R denotes an alkyl radical containing from 8 to 16 carbon atoms, G denotes glucose, fructose or galactose, and x is a value ranging from 1 to 4 and more particularly from 1 to 3.

45. Composition according to any one of the preceding claims, **characterized in that** the surfactant is an alkylpolyglucoside.

46. Composition according to any one of the preceding claims, **characterized in that** it also comprises one or more additional surfactants chosen from polycondensates of ethylene oxide and of propylene oxide on an alkyl chain; fatty acid esters of polyols; alkoxylated alkamides, polyoxyethylenated carboxylic acid salts, sarcosinates, glutamates, alkyl sulfates, alkyl ether sulfates, sulfonates, isethionates, taurates, sulfosuccinates, alkyl sulfoacetates, polypeptides and anionic alkyl polyglucoside derivatives, and mixtures thereof.

47. Composition according to any one of the preceding claims, **characterized in that** the total amount of surfactant (s) ranges from 0.1% to 50% by weight, of active material and preferably from 0.2% to 40% by weight of active material relative to the total weight of the composition.

48. Composition according to any one of the preceding claims, **characterized in that** it comprises at least one alkylpolyglycoside.

49. Composition according to the preceding claim, **characterized in that** the amount of alkylpolyglycoside(s) preferably represents from 0.1% to 100% and better still from 10% to 100% by weight relative to the total weight of surfactants.

50. Composition according to any one of the preceding claims, **characterized in that** the physiologically acceptable medium is constituted of water or of water and at least one organic solvent chosen from the group constituted of hydrophilic organic solvents, lipophilic organic solvents and amphiphilic solvents, or mixtures thereof.

51. Composition according to any one of the preceding claims, **characterized in that** it also comprises at least one oily phase.

52. Cosmetic use of a composition according to any one of the preceding claims, as cleansing Products and/or makeup-removing products for the skin, the scalp and/or the hair, and/or as shampoos and/or as scrubbing products and/or as exfoliant products for the skin.

53. Cosmetic process for cleansing the skin, the scalp and/or the hair, **characterized in that** the composition according to any one of Claims 1 to 51 is applied to the skin, to the scalp and/or to the hair, in the presence of water, and **in that** the foam formed and the soiling residues are removed by rinsing with water.

54. Use of the composition according to any one of Claims 1 to 51, for the preparation of a composition for treating greasy skin.

55. Use of an amphiphilic polymer comprising at least one ethylenically unsaturated monomer containing a sulfonic group in free form or in partially or totally neutralized form and comprising at least one hydrophobic part, to increase the volume of foam of a composition containing at least one surfactant.

## Patentansprüche

1. Reinigende Zusammensetzung, die in einem physiologisch akzeptablen, wässrigen Medium mindestens einen grenzflächenaktiven Stoff, der unter den Alkylpolyglycosiden, Maltoseestern, mehrfach mit Glycerin veretherten Fettalkoholen, N-Alkylglucaminderivaten, Amidoethercarboxylaten, Acetaten, Alaninaten, Aspartaten, Glycinaten, Citraten, Galacturonaten, Fettsäuresalzen, die Seifen bilden, Phosphaten und amphoteren und zwitterionischen grenzflächenaktiven Stoffen ausgewählt ist, und mindestens ein amphiphiles Polymer enthält, das mindestens ein Monomer mit ethylenisch ungesättigter Bindung und einer in freier Form oder ganz oder teilweise neutralisiert vorliegenden Sulfonsäuregruppe enthält und mindestens einen hydrophoben Bereich aufweist.

2. Zusammensetzung nach Anspruch 1, **dadurch gekennzeichnet, dass** der hydrophobe Teil des amphiphilen Polymers 6 bis 50 Kohlenstoffatome aufweist.

3. Zusammensetzung nach dem vorhergehenden Anspruch, **dadurch gekennzeichnet, dass** der hydrophobe Teil des amphiphilen Polymers 6 bis 22 Kohlenstoffatome besitzt.

4. Zusammensetzung nach dem vorhergehenden Anspruch, **dadurch gekennzeichnet, dass** der hydrophobe Teil des amphiphilen Polymers 6 bis 18 Kohlenstoffatome aufweist.

5. Zusammensetzung nach dem vorhergehenden Anspruch, **dadurch gekennzeichnet, dass** der hydrophobe Teil des amphiphilen Polymers 12 bis 18 Kohlenstoffatome besitzt.

6. Zusammensetzung nach einem der vorhergehenden Ansprüche, **dadurch gekennzeichnet, dass** die amphiphilen Polymere mit einer anorganischen oder organischen Base ganz oder teilweise neutralisiert sind.

7. Zusammensetzung nach einem der vorhergehenden Ansprüche, **dadurch gekennzeichnet, dass** die amphiphilen Polymere eine zahlenmittlere Molmasse von 1 000 bis 20 000 000 g/mol auf weisen.

8. Zusammensetzung nach dem vorhergehenden Anspruch, **dadurch gekennzeichnet, dass** die zahlenmittlere Molmasse im Bereich von 20 000 bis 5 000 000 g/mol liegt.

9. Zusammensetzung nach dem vorhergehenden Anspruch, **dadurch gekennzeichnet, dass** die zahlenmittlere Molmasse im Bereich von 100 000 bis 1 500 000 g/mol liegt.

10. Zusammensetzung nach einem der vorhergehenden Ansprüche, **dadurch gekennzeichnet, dass** eine wässrige Lösung der Polymere von 1 Gew.-% bei einer Temperatur von 25 °C eine mit dem Brookfield-Viskosimeter, Nadel 7, gemessene Viskosität von 20 000 bis 100 000 mPa·s aufweist.

11. Zusammensetzung nach einem der vorhergehenden Ansprüche, **dadurch gekennzeichnet, dass** die amphiphilen Polymere durch radikalische Polymerisation unter Fällen in *tert*-Butanol hergestellt sind.

12. Zusammensetzung nach einem der vorhergehenden Ansprüche, **dadurch gekennzeichnet, dass** die amphiphilen Polymere vernetzt oder nicht vernetzt sind.

13. Zusammensetzung nach dem vorhergehenden Anspruch, **dadurch gekennzeichnet, dass** die amphiphilen Polymere vernetzt sind.

14. Zusammensetzung nach dem vorhergehenden Anspruch, **dadurch gekennzeichnet, dass** das oder die Vernetzungsmittel unter den Verbindungen mit mehreren olefinisch ungesättigten Bindungen ausgewählt sind.

15. Zusammensetzung nach dem vorhergehenden Anspruch, **dadurch gekennzeichnet, dass** das oder die Vernetzungsmittel unter Methylen-bis-acrylamid, Allylmethacrylat oder Trimethylolpropantriacrylat (TMPTA) ausgewählt sind.

16. Zusammensetzung nach einem der Ansprüche 13 bis 15, **dadurch gekennzeichnet, dass** der Vernetzungsgrad vorzugsweise im Bereich von 0,01 bis 10 Mol-% und insbesondere 0,2 bis 2 Mol-%, bezogen auf das Polymer, liegt.

17. Zusammensetzung nach einem der vorhergehenden Ansprüche, **dadurch gekennzeichnet, dass** das Monomer mit ethylenisch ungesättigter Bindung und Sulfonsäuregruppe unter Vinylsulfonsäure, Styrolsulfonsäure, (Meth)acrylamido(C₁₋₂₂)alkylsulfonsäuren, N-(C₁₋₂₂)Alkyl(meth)acrylamido(C₁₋₂₂)alkylsulfonsäuren sowie deren ganz oder teilweise neutralisierten Formen ausgewählt ist.

18. Zusammensetzung nach dem vorhergehenden Anspruch, **dadurch gekennzeichnet, dass** das Monomer mit ethylenisch ungesättigter Bindung und Sulfonsäuregruppe unter Acrylamidomethansulfonsäure, Acrylamido-ethansulfonsäure, Acrylamidopropansulfonsäure, 2-Acrylamido-2-methylpropansulfonsäure, Methacrylamido-2-methylpropansulfonsäure, 2-Acrylamido-n-butansulfonsäure, 2-Acrylamido-2,4,4-trimethylpentansulfonsäure, 2-Methacrylamido-dodecylsulfonsäure, 2-Acrylamido-2,6-dimethyl-3-heptansulfonsäure sowie deren ganz oder teilweise neutralisierten Formen ausgewählt ist.

19. Zusammensetzung nach einem der Ansprüche 17 oder 18, **dadurch gekennzeichnet, dass** es sich bei dem Monomer mit ethylenisch ungesättigter Bindung und Sulfonsäuregruppe um die 2-Acrylamido-2-methylpropansulfonsäure (AMPS) sowie deren ganz oder teilweise neutralisierten Formen handelt.

20. Zusammensetzung nach dem vorhergehenden Anspruch, **dadurch gekennzeichnet, dass** die amphiphilen Polymere unter den statistischen Polymeren von AMPS ausgewählt sind, die durch Umsetzung mit einem *n*-Mono(C₆₋₂₂)alkylamin oder einem Di-*n-*(C₆₋₂₂)alkylamin modifiziert sind.

21. Zusammensetzung nach einem der Ansprüche 19 oder 20, **dadurch gekennzeichnet, dass** die amphiphilen AMPS-Polymere ferner mindestens ein Monomer mit ethylenisch ungesättigter Bindung enthalten, das keine Fettkette aufweist.

22. Zusammensetzung nach dem vorhergehenden Anspruch, **dadurch gekennzeichnet, dass** das Monomer mit ethylenisch ungesättigter Bindung, das keine Fettkette aufweist, unter (Meth)-acrylsäure und deren in β-Stellung Alkyl-substituierten Derivaten und ihren Estern, die mit Monoalkoholen oder Mono- oder Polyalkylenglycolen gebildet werden, oder den (Meth)acrylamiden, Vinylpyrrolidon, Maleinsäureanhydrid, Itaconsäure oder Maleinsäure oder den Gemischen dieser Verbindungen ausgewählt ist.

23. Zusammensetzung nach einem der vorhergehenden Ansprüche, **dadurch gekennzeichnet, dass** die amphiphilen Polymere unter den amphiphilen Copolymeren von AMPS und mindestens einem hydrophoben Monomer mit ethylenisch ungesättigter Bindung, das mindestens einen hydrophoben Teil mit 6 bis 50 Kohlenstoffatomen aufweist, ausgewählt sind.

24. Zusammensetzung nach dem vorhergehenden Anspruch, **dadurch gekennzeichnet, dass** der hydrophobe Teil 6 bis 22 Kohlenstoffatome aufweist.

25. Zusammensetzung nach dem vorhergehenden Anspruch, **dadurch gekennzeichnet, dass** der hydrophobe Teil 6 bis 18 Kohlenstoffatome aufweist.

26. Zusammensetzung nach dem vorhergehenden Anspruch, **dadurch gekennzeichnet, dass** der hydrophobe Teil 12 bis 18 Kohlenstoffatome aufweist.

27. Zusammensetzung nach einem der Ansprüche 23 bis 26, **dadurch gekennzeichnet, dass** das hydrophobe Monomer mit ethylenisch ungesättigter Bindung unter den Acrylaten oder Acrylamiden der folgenden Formel (I) ausgewählt ist 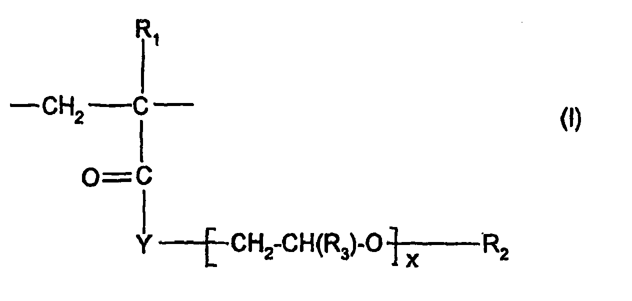 worin die Gruppen R₁ und R₃, die gleich oder verschieden sind, ein Wasserstoffatom oder eine geradkettige oder verzweigte C₁₋₆-Alkylgruppe (vorzugsweise Methyl) bedeuten; Y O oder N R₁ ist, wobei R₁ die oben angegebene Bedeutung aufweist; R₂ eine hydrophobe Kohlenwasserstoffgruppe mit mindestens 6 bis 50 Kohlenstoffatomen, vorzugsweise 6 bis 22 Kohlenstoffatomen, besonders bevorzugt 6 bis 18 Kohlenstoffatomen und insbesondere 12 bis 18 Kohlenstoffatomen bedeutet; und x die Molzahl an Alkylenoxid angibt und im Bereich von 0 bis 100 liegt.

28. Zusammensetzung nach dem vorhergehenden Anspruch, **dadurch gekennzeichnet, dass** die hydrophobe Gruppe R₂ unter den geradkettigen, verzweigten oder cyclischen C₆₋₁₈-Alkylgruppen; Alkyl-perfluorierten Gruppen mit 6 bis 18 Kohlenstoffatomen; der Cholesterylgruppe oder einem Cholesterinether; und aromatischen polycyclischen Gruppen ausgewählt ist.

29. Zusammensetzung nach einem der Ansprüche 27 oder 28, **dadurch gekennzeichnet, dass** das Monomer der Formel (I) ferner mindestens eine Alkylenoxideinheit (x ≥ 1) aufweist.

30. Zusammensetzung nach einem der Ansprüche 27 bis 29, **dadurch gekennzeichnet, dass** das Monomer der Formel (I) mindestens eine Polyoxyalkylenkette enthält.

31. Zusammensetzung nach dem vorhergehenden Anspruch, **dadurch gekennzeichnet, dass** die Polyoxyalkylenkette aus Ethylenoxideinheiten und/oder Propylenoxideinheiten besteht.

32. Zusammensetzung nach dem vorhergehenden Anspruch, **dadurch gekennzeichnet, dass** die Polyoxyalkylenkette ausschließlich aus Ethylenoxideinheiten besteht.

33. Zusammensetzung nach einem der Ansprüche 27 bis 32, **dadurch gekennzeichnet, dass** die Molzahl an Alkylenoxid im Bereich von 3 bis 100 liegt.

34. Zusammensetzung nach dem vorhergehenden Anspruch, **dadurch gekennzeichnet, dass** die Alkylenoxid-Molzahl im Bereich von 3 bis 50 liegt.

35. Zusammensetzung nach dem vorhergehenden Anspruch, **dadurch gekennzeichnet, dass** die Alkylenoxid-Molzahl im Bereich von 7 bis 25 liegt.

36. Zusammensetzung nach einem der Ansprüche 23 bis 28, **dadurch gekennzeichnet, dass** das amphiphile AMPS-Polymer ausgewählt ist unter:
- vernetzten oder nicht vernetzten, neutralisierten oder nicht neutralisierten Copolymeren, die 15 bis 60 Gew.-% AMPS-Einheiten und 40 bis 85 Gew.-% (C₈₋₁₆)Alkyl(meth)acrylamid-Einheiten oder (C₈₋₁₆)Alkyl(meth)acrylat-Einheiten, bezogen auf das Polymer, enthalten;
- Terpolymeren, die 10 bis 90 Mol-% Acrylamid, 0,1 bis 10 Mol-% AMPS-Einheiten und 5 bis 80 Mol-% *n*-(C₆₋₁₈)Alkylacrylamideinheiten, bezogen auf das Polymer, enthalten.

37. Zusammensetzung nach einem der Ansprüche 23 bis 28, **dadurch gekennzeichnet, dass** das amphiphile AMPS-Polymer ausgewählt ist unter:
- nicht vernetzten Copolymeren von ganz oder teilweise neutralisierter AMPS und *n*-Dodecylmethacrylat oder *n*-Hexadecylmethacrylat;
- vernetzten oder nicht vernetzten Copolymeren von ganz oder teilweise neutralisierter AMPS und *n*-Dodecylmethacrylamid.

38. Zusammensetzung nach den Ansprüchen 23 bis 35, **dadurch gekennzeichnet, dass** das amphiphile AMPS-Polymer unter den Copolymeren ausgewählt ist, die aus 2-Acrylamido-2-methylpropansulfonsäure-Einheiten (AMPS) der folgenden Formel (II) : 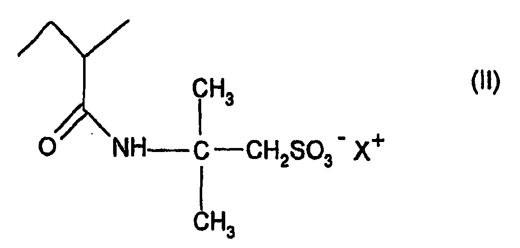 worin X⁺ ein Proton, ein Alkalimetallkation, ein Erdalkalimetallkation oder das Ammoniumion bedeutet, und Einheiten der folgenden Formel (III) bestehen: 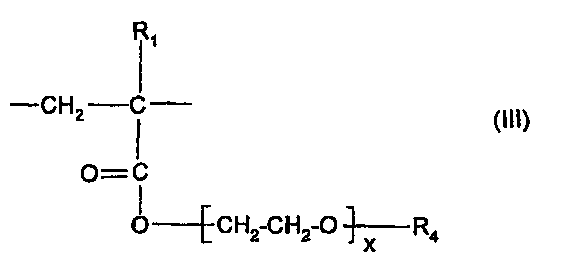 worin x eine ganze Zahl im Bereich von 3 bis 100, vorzugsweise 5 bis 80 und besonders bevorzugt 7 bis 25 ist; R₁ die oben in Formel (I) angegebene Bedeutung aufweist und R₄ eine geradkettige oder verzweigte C₆₋₂₂-Alkylgruppe und insbesondere C₁₀₋₂₂₋Alkylgruppe ist.

39. Zusammensetzung nach dem vorhergehenden Anspruch, **dadurch gekennzeichnet, dass** x = 25, R₁ bedeutet Methyl und R₄ ist *n*-Dodecyl, *n*-Hexadecyl oder n-Octadecyl.

40. Zusammensetzung nach Anspruch 27 oder 38, **dadurch gekennzeichnet, dass** der prozentuale Molanteil der Einheiten der Formel (I) oder der Einheiten der Formel (III) in den Polymeren im Bereich von 50,1 bis 99,9 % liegt.

41. Zusammensetzung nach Anspruch 27 oder 38, **dadurch gekennzeichnet, dass** der prozentuale Molanteil der Einheiten der Formel (I) oder der Einheiten der Formel (III) in den Polymeren im Bereich von 0,1 bis 50 % liegt.

42. Zusammensetzung nach einem der vorhergehenden Ansprüche, **dadurch gekennzeichnet, dass** der Mengenanteil des amphiphilen Polymers (oder der amphiphilen Polymere) im Bereich von 0,01 bis 50 Gew.-% wirksame Substanz, vorzugsweise 0,1 bis 20 Gew.-%, noch bevorzugter 0,2 bis 10 Gew.-% und insbesondere 0,25 bis 5 Gew.-% wirksame Substanz, bezogen auf das Gesamtgewicht der Zusammensetzung, liegt.

43. Zusammensetzung nach einem der vorhergehenden Ansprüche, **dadurch gekennzeichnet, dass** der grenzflächenaktive Stoff ein Alkylpolyglycosid ist, das durch die folgende allgemeine Formel (IV) dargestellt werden kann:
R-O-(G)ₓ (IV)
worin R eine geradkettige oder verzweigte, gesättigte oder ungesättigte Alkylgruppe mit 6 bis 30 Kohlenstoffatomen ist, G einen reduzierenden Zucker mit 5 bis 6 Kohlenstoffatomen bedeutet und x einen Wert von 1 bis 15 hat.

44. Zusammensetzung nach dem vorhergehenden Anspruch, **dadurch gekennzeichnet, dass** das Alkylpolyglycosid eine Verbindung der Formel (IV) ist, worin R eine Alkylgruppe mit 8 bis 16 Kohlenstoffatomen bedeutet, G Glucose, Fructose oder Galactose ist und x einen Wert von 1 bis 4 und insbesondere 1 bis 3 hat.

45. Zusammensetzung nach einem der vorhergehenden Ansprüche, **dadurch gekennzeichnet, dass** der grenzflächenaktive Stoff ein Alkylpolyglucosid ist.

46. Zusammensetzung nach einem der vorhergehenden Ansprüche, **dadurch gekennzeichnet, dass** sie ferner einen oder mehrere ergänzende grenzflächenaktive Stoffe enthält, die unter den Polykondensaten von Ethylenoxid und Propylenoxid und einer Alkylkette; Polyolfettsäureestern; alkoxylierten Alkamiden, polyethoxylierten Carbonsäuresalzen, Sarcosinaten, Glutamaten, Alkylsulfaten, Alkylethersulfaten, Sulfonaten, Isethionaten, Tauraten, Sulfosuccinaten, Alkylsulfoacetaten, Polypeptiden, anionischen Alkylpolyglucosidderivaten und deren Gemischen ausgewählt sind.

47. Zusammensetzung nach einem der vorhergehenden Ansprüche, **dadurch gekennzeichnet, dass** die Gesamtmenge des oder der grenzflächenaktiven Stoffe im Bereich von 0,1 bis 50 Gew.-% wirksame Substanz und vorzugsweise 0,2 bis 40 Gew.-% wirksame Substanz, bezogen auf das Gesamtgewicht der Zusammensetzung, liegt.

48. Zusammensetzung nach einem der vorhergehenden Ansprüche, **dadurch gekennzeichnet, dass** sie mindestens ein Alkylpolyglycosid enthält.

49. Zusammensetzung nach dem vorhergehenden Anspruch, **dadurch gekennzeichnet, dass** der Mengenanteil des oder der Alkylpolyglycoside vorzugsweise 0,1 bis 100 % und besser 10 bis 100 Gew.-%, bezogen auf das Gesamtgewicht der grenzflächenaktiven Stoffe, ausmacht.

50. Zusammensetzung nach einem der vorhergehenden Ansprüche, **dadurch gekennzeichnet, dass** das physiologisch akzeptable Medium aus Wasser oder Wasser und mindestens einem organischen Lösungsmittel besteht, das unter den hydrophilen organischen Lösungsmitteln, lipophilen organischen Lösungsmitteln, amphiphilen Lösungsmitteln oder deren Gemischen ausgewählt ist.

51. Zusammensetzung nach einem der vorhergehenden Ansprüche, **dadurch gekennzeichnet, dass** sie ferner mindestens eine Ölphase enthält.

52. Kosmetische Verwendung einer Zusammensetzung nach einem der vorhergehenden Ansprüche als Produkt für die Reinigung und/oder zum Abschminken der Haut, der Kopfhaut und/oder der Haare und/oder als Haarwaschmittel und/oder als abradierendes Produkt und/oder als exfoliatives Produkt für die Haut.

53. Kosmetisches Verfahren zur Reinigung der Haut, der Kopfhaut und/oder der Haare, **dadurch gekennzeichnet, dass** die Zusammensetzung nach einem der Ansprüche 1 bis 51 in Gegenwart von Wasser auf die Haut, die Kopfhaut und/oder auf die Haare aufgebracht und der gebildete Schaum und die Schmutzrückstände durch Spülen mit Wasser entfernt werden.

54. Verwendung der Zusammensetzung nach einem der Ansprüche 1 bis 51 für die Herstellung einer Zusammensetzung, die zur Behandlung von fettiger Haut vorgesehen ist.

55. Verwendung eines amphiphilen Polymers, das mindestens ein Monomer mit ethylenisch ungesättigter Bindung und einer Sulfonsäuregruppe in freier Form oder ganz oder teilweise neutralisiert enthält und mindestens einen hydrophoben Bereich aufweist, um das Schaumvolumen einer Zusammensetzung, die mindestens einen grenzflächenaktiven Stoff enthält, zu erhöhen.
